# EUROPEAN PATENT SPECIFICATION

(11) **EP 4 392 109 B1**
(45) Date of publication and mention of the grant of the patent: **30.09.2026**
(21) Application number: 22861787.4
(22) Date of filing: 26.08.2022
(51) Int. Cl.: A61M 16/04, A61M 16/08

(54) **TRACHEOSTOMY GUARD AND ASSOCIATED ASSEMBLY**
TRACHEOSTOMIESCHUTZ UND ZUGEHÖRIGE ANORDNUNG
PROTECTION POUR TRACHÉOSTOMIE ET ENSEMBLE ASSOCIÉ

(30) Priority: 27.08.2021 US 202163260665 P
(43) Date of publication of application: 03.07.2024
(60) Divisional application: 26199947.8
(73) Proprietor: Fisher & Paykel Healthcare Limited, Auckland, 2013 (NZ)
(72) Inventor: VAN SCHALKWYK, Andre, Auckland, 2013 (NZ); O'DONNELL, Kevin Peter, Auckland, 2013 (NZ)
(74) Representative: Ström & Gulliksson AB
(86) International application number: PCT/NZ2022/050114
(87) International publication number: WO 2023/027601

(56) References cited:
- EP-A1- 1 693 081
- WO-A1-2006/072768
- WO-A1-2011/122964
- WO-A1-2021/099470
- DE-U1- 202004 004 633
- US-A1- 2003 037 789
- US-A1- 2004 123 868
- US-A1- 2018 078 723
- US-A1- 2019 290 876
- US-A1- 2020 188 620
- US-B2- 10 828 445

## Description

### TECHNICAL FIELD

The present disclosure generally relates to respiratory assistance systems. More particularly, the present disclosure relates to accessories and/or patient interfaces for providing respiratory therapy to a patient.

### BACKGROUND

A tracheostomy is an opening surgically made into the trachea of a patient to provide an airway and remove secretions from the lungs. Respiratory gases may be provided from a gases source to the patient via a patient interface, such as a tracheostomy interface. The tracheostomy interface may be connected to a port formed in the tracheostomy to facilitate ventilation.

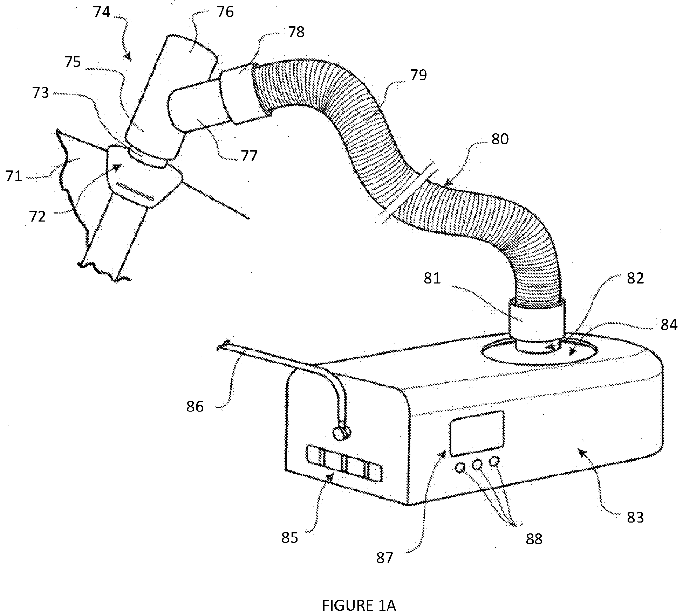

Tracheal couplings (for example in the form of connectors of trachea interfaces) may be used in conjunction with a conventional respiratory assistance system such as that shown in Figure 1a. A tracheal coupling provides for passage of breathing gases (such as oxygen) interfacing between a high flow gases supply and a patient (such as a tracheotomised or intubated patient.) A tracheal coupling can take the form of a connector that couples to a trachea insert/interface in or on a tracheotomised or intubated patient. In one example a trachea interface can be attached to a tracheotomised/intubated patient.

A tracheostomy guard may be coupled to the trachea interface to prevent occlusion of the patient interface, for example, by the chin of a patient, bedding or dressings. The tracheostomy guard comprises openings to allow air exchange therein.

During tracheal ventilation, patient secretions may be expelled from a patient interface. The secretions may be communicated to a caregiver, for example, through openings in a tracheostomy guard, or directly from the interface. Secretions can exit the openings suddenly and with enough force to reach a caregiver positioned near a patient. A tracheostomy guard may facilitate the removal of secretions therefrom while deflecting secretions such that they are not directed to a caregiver.

Provision of high flow therapy may be used to flush airways and reduce deadspace, and produce positive expiratory end pressure (PEEP), improved humidification to promote mucocillary transfer within the lungs. In some instances of use high flow therapy can also provide dynamic positive pressure. However, conventional tracheostomy guards may not be suitable for such a high gas flow environment. An improved tracheostomy guard for high flow tracheostomy would therefore be advantageous.

US 2003/037789 A1 discloses a trachestomy collar suitable for removing secretions. US 2018/078723 A1 discloses an airway device for controlling respiratory secretions in artificial airways.

### SUMMARY

The present disclosure relates to a tracheostomy guard (hereinafter also referred to as a "trache guard") to be used with a tracheostomy interface (hereinafter also referred to as a "trache interface" or "patient interface").

The invention is defined in the appended independent claim 1. Preferred embodiments are matter of the dependent claims.

In a first aspect there may be provided a tracheostomy guard for a patient interface, comprising: a first portion arranged to connect to a patient interface in fluid communication with the first portion, in use, a second portion in fluid communication with the first portion and comprising at least one first member and/or second member forming at least one aperture in fluid communication with the ambient environment, wherein the aperture has a size variable in response to a force applied to at least part of the second portion, in use.

In some embodiments the at least one first member is arranged to deflect or move bidirectionally relatively the second member in response to the force applied.

In some embodiments the size of the aperture relates to a cross-sectional dimension of the aperture.

In some embodiments the force applied is associated with a gases flow rate of gases in the tracheostomy guard.

In some embodiments the gases flow rate relates to a high flow rate of 20l/min to 150l/min or 20l/min to 80l/min for adult patients, and 1l/min to 30l/min for neonatal or paediatric patients.

In some embodiments the variable size of the aperture is configured to allow for sputum release therethrough in response to the force applied, in use.

In some embodiments the force applied to the second portion in use is due at least part to a weight of sputum present in the second portion.

In some embodiments the variable size of the aperture is configured to achieve a set minimum positive end expiratory pressure (PEEP), in use.

In some embodiments the set minimum PEEP is selected from a range of: 1cmH₂0 to 7cmH₂0.

In some embodiments, the at least one first member and/or the second member are relatively arranged to form the at least one variable sized aperture.

In some embodiments, the first member(s) has a first material or structural characteristic, and the second member(s) has a second material or structural characteristic, wherein the first material or structural characteristic and the second material or structural characteristic are selected to allow the size of the variable sized aperture to change in response to the applied force.

In some embodiments, the first material or structural characteristic and second material or structural characteristic relates to at least one of the following: rigidity, stiffness, flexibility, modulus of elasticity, second moment of area, length, width, and/or thickness.

In some embodiments, the first material or structural characteristic relates to a thickness of the first member and/or the second material or structural characteristic relates to a thickness of the second member.

In some embodiments the at least one first member or second member is made of a polymer, an elastomer, a thermoplastic polymer or elastomer, or a thermoset polymer.

In some embodiments the second portion is releasably attached to the first portion.

In some embodiments the second portion is sealingly attached to the first portion.

In some embodiments at least a part of the second portion is integral with the first portion.

In some embodiments the force applied is associated with an internal pressure of said second portion.

In some embodiments the first portion and second portion together form a cavity that is in fluid communication with the ambient environment only via the variable sized aperture(s), in use.

In some embodiments the aperture attains its smallest size in a resting state of the tracheostomy guard.

In some embodiments the at least one first member or second member comprises a flap or panel.

In some embodiments the first material or structural characteristic and/or second material or structural characteristic is selected to enable reversible deformation or deflection or bend or flex of the first member and/or second member based on the applied force.

In some embodiments the size of aperture is variable based on the reversible deformation.

In some embodiments, said second portion is configured to be in fluid communication with the patient interface, in use.

In a second aspect there may be provided a tracheostomy guard for a patient interface, comprising: a first portion arranged to attach to a patient interface in fluid communication with the first portion, in use, a second portion in fluid communication with the first portion. The second portion comprises at least one first member having a first material or structural characteristic, and a second member having a second material or structural characteristic, wherein the first material or structural characteristic is different from the second material or structural characteristic and is selected to allow for reversible deformation or deflection or bend or flex of the first member in response to force applied to at least part of the second portion, in use.

In some embodiments the at least one first member and/or second member are relatively arranged to form at least one aperture in fluid communication with the ambient environment, wherein the aperture has an effective size variable in response to the applied force acting on the first member, in use.

In some embodiments, the at least one first member is arranged to deflect or move relative the second member in response to the force applied.

In some embodiments, the size of the aperture relates to a cross-sectional dimension of the aperture.

In some embodiments, the force applied is associated with a flow rate of gases in the tracheostomy guard.

In some embodiments, the gases flow rate relates to a high flow rate of 20l/min to 150l/min or 20l/min to 80l/min for adult patients, and 1l/min to 30l/min for neonatal or paediatric patients.

In some embodiments, the variable size of the aperture is configured to allow for sputum release therethrough in response to the force applied, in use.

In some embodiments, the force applied to the second portion in use is due at least part to a weight of sputum present in the second portion.

In some embodiments, the variable size of the aperture is configured to achieve a set minimum positive end expiratory pressure (PEEP), in use.

In some embodiments, set minimum PEEP is selected from a range of: 1cmH₂0 to 7cmH₂0.

In some embodiments, the first material or structural characteristic and second material or structural characteristic relates to at least one of the following: rigidity, stiffness, flexibility, modulus of elasticity, second moment of area, length, width, and/or thickness.

In some embodiments, the first material or structural characteristic relates to a thickness of the first member(s) and/or the second material or structural characteristic relates to a thickness of the second member.

In some embodiments, the at least one first member or second member is made of a polymer, an elastomer, a thermoplastic polymer or elastomer, or a thermoset polymer.

In some embodiments, the second portion is releasably attached to the first portion.

In some embodiments, the second portion is sealingly attached to the first portion.

In some embodiments, at least a part of the second portion is integral with the first portion.

In some embodiments, the force applied is associated with an internal pressure of said second portion.

In some embodiments, the first portion and second portion together form a cavity that is in fluid communication with the ambient environment only via the variable sized aperture(s), in use.

In some embodiments, the aperture attains its smallest size in a resting state of the tracheostomy guard.

In some embodiments, the first member or second member comprises a flap or panel.

In some embodiments, aperture size varies based on the reversible deformation.

In a third aspect there may be provided a tracheostomy guard for a patient interface, comprising: a first portion having an end arranged to releasably attach to a patient interface, the first portion comprising an alignment feature having an exterior shape or boundary for alignment with a corresponding alignment feature of the patient interface when connected to the tracheostomy guard, and an attachment member arranged to: mate with a corresponding attachment member of the patient interface when the first portion is attached to the patient interface, and disconnect from the corresponding attachment member of the patient interface upon the application of a directional force to the first portion.

In some embodiments the alignment feature relates to an exterior shape or boundary of the first portion.

In some embodiments, the alignment feature relates to an exterior shape or boundary of an oblong or tongue shaped extension of the first portion.

In some embodiments, the attachment member comprises an attachment release lip.

In some embodiments, the attachment member is formed by a smooth tubular interior surface of the first portion, wherein the smooth tubular interior surface is arranged to mate with a corresponding smooth exterior surface of the patient interface.

In some embodiments, the attachment member is arranged to mate with the corresponding attachment member of the patient interface by means of a friction fit.

In some embodiments, the attachment member comprises a protrusion configured to mate with a corresponding recess of the patient interface.

In some embodiments, the attachment member comprises a recess configured to mate with a corresponding protrusion of the patient interface.

In some embodiments, the attachment member is configured to sealingly attach to the corresponding attachment member of the patient interface.

In a fourth aspect there may be provided an assembly for providing high flow tracheostomy, including a tracheostomy guard as disclosed herein, and at least one of the following: a tracheostomy patient interface for connection to the tracheostomy guard, a conduit or tube arranged to connect a gases source at a first end thereof and to the tracheostomy patient interface at a second end thereof, and a gases source supplying for supplying a flow of gas to the patient interface via the conduit, in use.

In some embodiments, the gases source comprises a blower and humidifier.

In some embodiments, the conduit or tube is a breathable tube that allows excess water vapour to move out of the tube to atmosphere.

In some embodiments, the tracheostomy interface comprises the breathable tube.

In a fifth aspect there may be provided a tracheostomy interface assembly for providing high flow tracheostomy, comprising a tracheostomy guard as disclosed herein, and a tracheostomy interface (20) comprising a first end for connection to an airway of a patient, a port for connection to a gases source for supplying a flow of gas to the patient via the first end, and a second end for connection to a first portion of the tracheostomy guard.

In some embodiments, the tracheostomy guard is releasably attachable to the second end of the tracheostomy interface via the first portion.

In a sixth aspect there may be provided a tracheostomy guard for a patient interface, the tracheostomy guard comprising: a first portion arranged to connect to a patient interface, in use; a second portion attached to the first portion and configured to be in fluid communication with the patient interface, in use, the second portion forming at least one aperture in fluid communication with an ambient environment, wherein the aperture has an effective size that is variable in response to a force applied to at least part of the second portion, in use.

In some embodiments, said at least part of the second portion comprises a region of the second portion proximate the aperture, said region being configured to resiliently deform such that the effective size of the aperture varies in response to applied force to said region.

In some embodiments, said region being resiliently deformable being a bidirectional manner.

In some embodiments, said region is biased towards a resting position at which the effective size of the aperture is at its smallest.

In some embodiments, said region is configured such that an increase in effective size occurs in a controlled or gradual manner, to enable a desired pressure to be maintained and prevent forceful expulsion of sputum.

In some embodiments, said region is configured such that, when starting from a or said resting position, the effective size of the aperture increases as the absolute value of said force applied increases.

In some embodiments, said second portion comprises at least one first member and/or second member; and said at least one first member and/or second member form said at least one aperture.

In some embodiments, said at least one first member and/or second member of said second portion is resiliently movable in response to the force applied to said at least part of the second portion, thereby providing said at least one aperture with a variable effective size.

In some embodiments, said at least one first member and/or second member of the second portion is resiliently movable in a bi-directional manner.

In some embodiments, the at least one first member is arranged to deflect or move relative to the at least one second member in response to the force applied.

In some embodiments, the effective size of the aperture is related to a cross-sectional dimension of the aperture.

In some embodiments, the force applied is associated with a gases flow rate of gases in the tracheostomy guard.

In some embodiments, the gases flow rate relates to a high flow rate of 20l/min to 150l/min or 20l/min to 80l/min for adult patients, and 1l/min to 30l/min for neonatal or paediatric patients.

In some embodiments, the variable effective size of the aperture is configured to allow for sputum release through the aperture in response to the force applied, in use.

In some embodiments, the force applied is due at least part to a weight of sputum present in the second portion.

In some embodiments, the variable size of the aperture is configured to achieve a set minimum positive end expiratory pressure (PEEP), in use.

In some embodiments, set minimum PEEP is selected from a range of: 1cmH₂0 to 7cmH₂0.

In some embodiments, the at least one first member and second member are relatively arranged to form the at least one variable sized aperture.

In some embodiments, the first member(s) has a first material or structural characteristic, and the second member(s) has a second material or structural characteristic, wherein the first material or structural characteristic and the second material or structural characteristic are selected to allow the size of the variable sized aperture to change in response to the applied force.

In some embodiments, the first material or structural characteristic and second material or structural characteristic relates to at least one of the following: rigidity, stiffness, flexibility, modulus of elasticity, second moment of area, length, width, and/or thickness.

In some embodiments, the first material or structural characteristic relates to a thickness of the first member and/or the second material or structural characteristic relates to a thickness of the second member.

In some embodiments, the first member and/or second member is made of a polymer, an elastomer, a thermoplastic polymer or elastomer, or a thermoset polymer.

In some embodiments, the second portion is releasably attached to the first portion.

In some embodiments, the second portion is sealingly attached to the first portion.

In some embodiments, at least a part of the second portion is integral with the first portion.

In some embodiments, the force applied is associated with an internal pressure within said second portion.

In some embodiments, the applied force is associated with a difference between the internal pressure and an ambient pressure externally of the second portion

In some embodiments, the second portion forms a cavity that is in fluid communication with the ambient environment only via the at least one variable sized aperture, in use.

In some embodiments, the aperture attains its smallest effective size in a resting state of the tracheostomy guard.

In some embodiments, the first member and/or second member comprises a flap or panel.

In some embodiments, the first material or structural characteristic and/or second material or structural characteristic is selected to enable reversible deformation of the first member and/or second member based on the applied force.

In some embodiments, the effective size of aperture is variable based on the reversible deformation.

In some embodiments, the attachment member is configured to sealingly attach to the corresponding attachment member of the patient interface.

In a seventh aspect there may be provided a valve unit for use with a tracheostomy guard, comprising: a hollow body having: a first port for connection to a corresponding port of a tracheostomy guard and a second port in fluid communication with the ambient environment, at least one first member and a second member forming at least one aperture, wherein the aperture has an effective size that is variable in response to a force applied to the first member and/or second member, in use.

In some embodiments, said aperture of said valve component is configured to coincide, in use, with an aperture on the second portion of the tracheostomy guard, when the valve component is substantially mated with the second portion.

In an eighth aspect there may be provided a valve component for use with a tracheostomy guard for a patient interface, the tracheostomy guard comprising: a first portion arranged to connect to a patient interface, in use; and a second portion attached to the first portion and configured to be in fluid communication with the patient interface, in use, the valve component being configured to substantially mate with the second portion and provide at least one aperture in fluid communication with an ambient environment, wherein the aperture has an effective size that is variable in response to applied force, in use.

In some embodiments, said aperture of said valve component is configured to coincide, in use, with an aperture on the second portion of the tracheostomy guard, when the valve component is substantially mated with the second portion.

In a ninth aspect, there may be provided a kit of parts, comprising: a tracheostomy guard as disclosed herein and at least one of the following: a tracheostomy patient interface for connection to the tracheostomy guard; a conduit or tube arranged to connect a gases source at a first end thereof and to the tracheostomy patient interface at a second end thereof; and a gases source for supplying a flow of gas to the patient interface via the conduit or tube, in use.

In some embodiments, the kit of parts comprises a tracheal tube coupled or couplable to the tracheostomy patient interface.

In some embodiments, the kit of parts comprises a connector for connecting the tracheal tube to the tracheostomy patient interface.

In some embodiments, said kit of parts is configured to provide a tracheostomy interface assembly for providing high flow therapy.

In a tenth aspect there may be provided a kit of parts, comprising: a tracheostomy guard; and a valve unit as disclosed herein or a valve component as disclosed herein, for use with said tracheostomy guard and at least one of the following: a tracheostomy patient interface for connection to the tracheostomy guard; a conduit or tube arranged to connect a gases source at a first end thereof and to the tracheostomy patient interface at a second end thereof; and a gases source for supplying a flow of gas to the patient interface via the conduit or tube, in use.

In some embodiments, the kit of parts comprises a tracheal tube coupled or couplable to the tracheostomy patient interface.

In some embodiments, the kit of parts comprises a connector for connecting the tracheal tube to the tracheostomy patient interface.

In some embodiments, said kit of parts is configured to provide a tracheostomy interface assembly for providing high flow therapy.

### BRIEF DESCRIPTION OF THE DRAWINGS

A number of embodiments will now be shown, by way of example, with reference to the following drawings, in which:
Figure 1a illustrates a respiratory assistance system of the prior art;
Figure 1b illustrates a perspective view of a tracheostomy connector and associated breathing tube of the prior art connected to a tracheotomy of a patient;
Figure 1c illustrates in diagrammatic form an example of a respiratory support apparatus;
Figure 1d shows a perspective view of an example of a respiratory support apparatus;
Figures 2 to 5 illustrate respective perspective views of a tracheostomy guard according to a first embodiment;
Figure 6 illustrates a bottom plan view of the tracheostomy guard of Figures 2 to 5;
Figure 7 illustrates an enlarged section of the tracheostomy guard of Figure 6;
Figure 8 illustrates a front plan view of the tracheostomy guard of Figures 2 to 7;
Figure 9 illustrates a back plan view of the tracheostomy guard of Figures 2 to 8;
Figure 10a illustrates a sectional view of the tracheostomy guard of the first embodiment in a resting state;
Figure 10b illustrates a sectional view of the tracheostomy guard of Fig 10a, when a portion of the tracheostomy guard is subject to a first applied force and/or interior pressure;
Figure 10c illustrates a sectional view of the tracheostomy guard of Fig 10a and 10b, when the portion of the tracheostomy guard is subject to a second applied force and/or interior pressure;
Figure 11 illustrates a sectional perspective view of the tracheostomy guard of Figures 2 to 9;
Figure 12 illustrates a further sectional perspective view of the tracheostomy guard of Figures 2 to 9;
Figures 13 and 14 illustrate respective perspective view of a patient interface according to the prior art;
Figures 15 to 17a illustrate respective perspective views of the tracheostomy guard of Figures 2 to 12 connected to the patient interface of Figures 13 to 14;
Figure 17b illustrate a perspective view of the tracheostomy guard of Figures 2 to 12 upon disconnection from the patient interface of Figures 13 to 14;
Figures 18 and 19 illustrate respective perspective views of a tracheostomy guard according to a second embodiment;
Figures 20 to 21 illustrate respective perspective views of the tracheostomy guard of the second embodiment when connected to the patient interface of Figures 13 and 14;
Figure 22 illustrates a bottom plan view of the tracheostomy guard of the second embodiment when connected to the patient interface of Figures 13 and 14;
Figures 23 to 26 illustrate respective perspective views of a tracheostomy guard according to a third embodiment;
Figure 27 illustrates a perspective view of the tracheostomy guard of the third embodiment when connected to the patient interface of Figures 13 to 14;
Figure 28 illustrates a front plan view of the tracheostomy guard of the third embodiment when connected to the patient interface of Figures 13 to 14;
Figure 29 illustrates a rear plan view of the tracheostomy guard of the third embodiment when connected to the patient interface of Figures 13 to 14;
Figure 30 illustrates a rear sectional view of the tracheostomy guard of the third embodiment when connected to the patient interface of Figures 13 to 14;
Figures 31 to 34 illustrate respective perspective views and bottom plan views of a tracheostomy guard according to a fourth embodiment;
Figure 35 illustrates a perspective view of the tracheostomy guard of the fourth embodiment when connected to the patient interface of Figures 13 to 14;
Figure 36 illustrates a front plan view of the tracheostomy guard of the fourth embodiment when connected to the patient interface of Figures 13 to 14;
Figure 37 illustrates a rear plan view of the tracheostomy guard of the fourth embodiment when connected to the patient interface of Figures 13 to 14;
Figures 38 to 42 illustrate respective perspective views and bottom plan views of a tracheostomy guard according to a fifth embodiment;
Figure 42 illustrates a perspective view of the tracheostomy guard of the fifth embodiment when connected to the patient interface of Figures 13 to 14;
Figure 43 illustrates a front plan view of the tracheostomy guard of the fifth embodiment when connected to the patient interface of Figures 13 to 14;
Figure 44 illustrates a rear plan view of the tracheostomy guard of the fifth embodiment when connected to the patient interface of Figures 13 to 14; and
Figure 45 illustrates perspective view of an assembly comprising the tracheostomy guard of the first embodiment, a tube, and patient interface.

### DETAILED DESCRIPTION

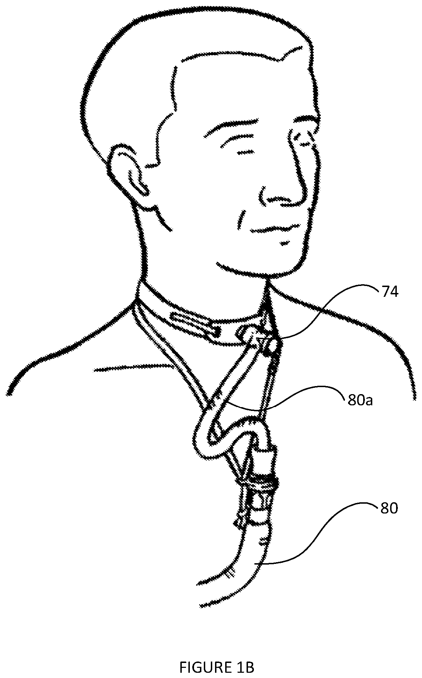

Figure 1a illustrates a prior art respiratory support system into which tracheal couplings, such as the tracheostomy guard(s) disclosed herein may be incorporated. An external portion to a tracheostomy tube 72 extends out of the neck of patient 71. A male connector 73 extends from the tracheostomy tube 72. A trachea connector 74 includes a patient end 75 connected to the male connector 73 of the tracheostomy tube. The connector 74 includes outlet end 76 and an inlet tube 77. The inlet tube is connected to the cuff 78 of a breathing tube 80. The breathing tube 80 includes a flexible conduit 79 for supplying breathing gases to the patient. At least a section 80a of the breathing tube 80 proximal to the patient, such as that identified with reference to Figure 1b, may be a breathable tube being made from a breathable material. A cuff 81 at the other end of the breathing tube 80 is connected to an outlet connector of a gases flow source. The gases flow source may be a flow generator 83, typically forming part of a respiratory support apparatus arranged to provide the patient with a respiratory gases flow.

The outlet end 76 of the connector 74 may be arranged to be connected to a conventional tracheostomy guard. Such a conventional tracheostomy guard is arranged to allow fluid at the outlet end 76 to exit the tracheostomy guard though a fixed size aperture during gas flow therapy or at least allow a clinician to suction out fluid through the fixed size aperture. As high flow therapy be carried out over a relatively wide range of flow rates (see below), it may be difficult using a conventional tracheostomy guard to keep the PEEP within suitable limits throughout the range of flow rates used for the high flow therapy. Further, conventional tracheostomy guards may also be prone to blockage when used during high flow therapy.

The flow generator 83 may optionally include a humidification system/humidifier including humidification chamber 84. The flow generator 83 may include an outlet connector 82 that is configured to connect to the cuff 81. Breathing gases can be delivered to the user at, or near, optimal temperature and humidity (37°C, 44 mg/L humidity) as the gases are delivered. Emulating the conditions within healthy adult lungs (37°C, 44 mg/L humidity) can help maintain healthy mucociliary function in users with respiratory disorders affecting secretion. Using a humification system/humidifier when providing trache therapy may be advantageous as the upper airways are bypassed. Further, a humidified gases flow makes high flow therapy more comfortable and tolerable for patients.

The flow generator 83 typically includes a blower receiving air from an air intake 85 and optionally oxygen from oxygen supply line 86. A user interface may include a display screen 87 and user controls 88. The user controls may be in the form of buttons on the housing of the flow generator or combined with the display screen as a touch screen on the flow generator. An example flow generator for use in this application is the Fisher & Paykel Healthcare AIRVO 2^{™} or the AIRVO 3.

The flow generator 83 includes a flow sensor and a feedback control which monitors the delivered flow and varies the blower speed to maintain the output flow of the generator at a level set through the user controls.

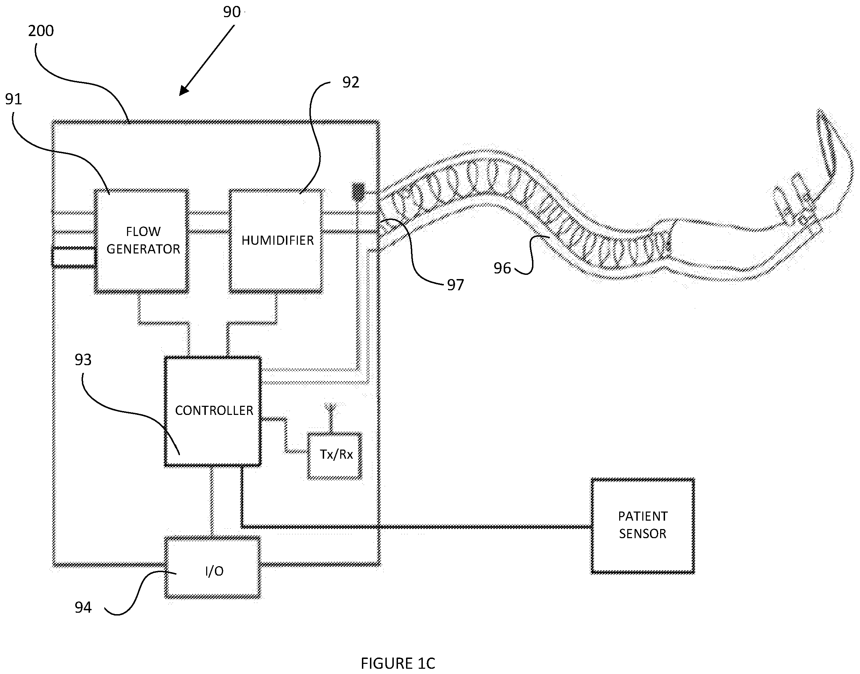

An example of a respiratory support apparatus 90 is shown in Figure 1c. The respiratory support apparatus 90 can comprise a housing 200 (for example as a single housing) that contains one or more of: a flow generator 91, which in some configurations is in the form of a motor/impeller arrangement (for example, a blower), a humidifier 92 pneumatically connected to the flow generator 91, a controller 93, and a user interface 94 (comprising, for example, a display and input device(s) such as button(s), a touch screen, or the like).

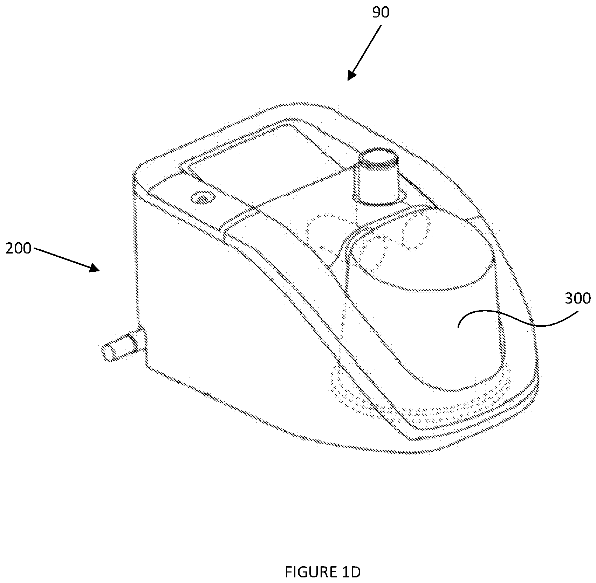

Figure 1d also shows the respiratory support apparatus 90 comprising the housing 200 and illustrates that the components of the respiratory support apparatus 90 (e.g., including the humidifier 92 being pneumatically connected to the flow generator 91) are integrated into a common housing. This provides a compact device that can be easily moved around or carried to provide mobility. Further the components of the respiratory support apparatus 90 (e.g., flow generator 91 and humidifier 92) being combined in the same housing allows for simpler set up (i.e., chamber 300 is positioned in the housing).

In some configurations, the respiratory support apparatus 90 may not comprise a flow generator 91. In this case the respiratory support apparatus 90 does not generate a flow of gases, and instead is configured to be connected to an external flow generator and configured to humidify the flow of gases from the external flow generator. For example, the respiratory support apparatus 90 can be used as a stand-alone humidifier to humidify gases flowing through the humidifier. The flow generator may be a wall gas supply (regulated via a flowmeter or rotameter, for example) or a ventilator or other separate flow generator that can be configured to provide high flow therapy (e.g. for NIV, CPAP, BCPAP, etc). The humidifier may include a battery coupled to the humidifier to supply power when mains is unavailable (as a battery supply). In some configurations, the battery may be removably coupled to the apparatus and is rechargeable. The humidifier is pneumatically coupled to a flow generator via a conduit and a separate conduit is coupled to the humidifier to convey humidified gases from the humidifier to a patient.

In some examples, a breathing conduit 96 is coupled at one end to a gases outlet 97 in the housing 200 of the respiratory support apparatus 90. The breathing conduit 96 is coupled at another end to a patient interface, which for present purposes is provided by a tracheostomy interface and/or tracheal coupling (e.g., the tracheostomy guard disclosed herein). As will be explained in further detail below, it should be appreciated that the combination of the tracheostomy interface and the tracheal coupling can be non-sealing (i.e., comprise a leak) in order to generate/provide a flushing effect (explained further below) and reduce risks of barotrauma.

Tracheal couplings, e.g. the tracheostomy guard disclosed herein and associated patient interface(s), may be used in providing high flow therapy where the patient is spontaneously breathing. This means that a substantial proportion of the air supplied to the connector vents directly to the room without being breathed by the patient. As will be further explained below, the tracheostomy guard may for example be connected to a tracheostomy or trache interface as indicated by connector 74 in Figures 1a or 1b. While a flow generator 83 such as that shown in Figure 1a may be used as gases source for providing the high flow of gases, in some configurations other high flow gases sources, e.g., a supply of air from a wall outlet or ventilator may be used. It should be appreciated that the tracheostomy guard disclosed herein is not limited for use with any particular type of gases source.

As mentioned previously, high gas flow environments, for example, in high flow therapy, may be used to flush airways and reduce deadspace, and produce positive expiratory end pressure (PEEP), and optionally to produce an elevated pressure in the patient airway. A positive end expiratory pressure (PEEP) can keep the airways and alveoli from collapsing at end-expiration and reopen airways and alveoli that have already collapsed. The therapeutic provision of PEEP can improve gas exchange (decreased intra pulmonary shunt), reduce the resistance to airflow (lung resistance), and make the lungs less stiff (increased lung compliance). Levels of oxygen and carbon dioxide also may improve due to high flow gases delivery, reducing the need for supplemental oxygen and the sensation of breathlessness. High flow therapy can also improve humidification within the lungs thereby improving mucociliary transport. PEEP may assist in the treatment of obstructive lung diseases and heart failure, including emphysema, bronchiectasis, chronic bronchitis, cystic fibrosis and pulmonary edema.

High flow therapy as discussed herein is intended to be given its typical ordinary meaning as understood by a person of skill in the art which generally refers to a breathing assistance apparatus delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen litres per minute to about sixty litres per minute or greater. Typical flow rates for paediatric patients (such as neonates, infants and children) often range from, but are not limited to, about one litre per minute per kilogram of patient weight to about three litres per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names. It will be understood that the present invention is particularly concerned with delivery of high flow therapy via a tracheostomy interface or tracheal coupling.

For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient, 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may, in some configurations, deliver gases to the patient at a flow rate of between about 1 LPM and about 100 LPM, or at a flow rate in any of the sub-ranges outlined above. Gases delivered may comprise a percentage of oxygen. In some configurations, the percentage of oxygen in the gases delivered may be between about 20% and about 100%, or between about 30% and about 100%, or between about 40% and about 100%, or between about 50% and about 100%, or between about 60% and about 100%, or between about 70% and about 100%, or between about 80% and about 100%, or between about 90% and about 100%, or about 100%, or 100%.

High flow therapy may be effective in meeting or exceeding the patient's inspiratory flow, increasing oxygenation of the patient, and/or reducing the work of breathing.

High flow therapy may be administered to the nares of a patient and/or orally, or via a tracheostomy interface.

High flow therapy may generate a flushing effect in the airways such that the anatomical dead space of the airways is flushed by the high incoming gases flow. This can create a reservoir of fresh gas available for each and every breath, while reducing re-breathing of nitrogen and carbon dioxide. High flow therapy can be delivered with a non-sealing patient interface such as, for example, tracheostomy interface (which may be coupled to a tracheal coupling and/or a tracheostomy guard. High flow therapy may slow down respiratory rate of the patient. High flow therapy may provide expiratory resistance to a patient.

High flow therapy may be used to treat patients with obstructive pulmonary conditions e.g., COPD, bronchiectasis, dyspnea, cystic fibrosis, emphysema and/or patients with respiratory distress or hypercapnic patients.

The term "non-sealing patient interface" (i.e., unsealed patient interface) as used herein can refer to an interface providing a pneumatic link between an airway of a patient and a gases flow source (such as from flow generator 91) that does not completely occlude the airway of the patient. A non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or both nares of the patient and/or their mouth. For a tracheostomy interface, the airway is through the trachea.

As will be further elucidated below, the tracheostomy guard disclosed in various embodiments herein is designed for such high gas flow environments.

The tracheostomy guard may allow for producing a desired PEEP pressure while still having a desired flushing effect at high associated flow rates.

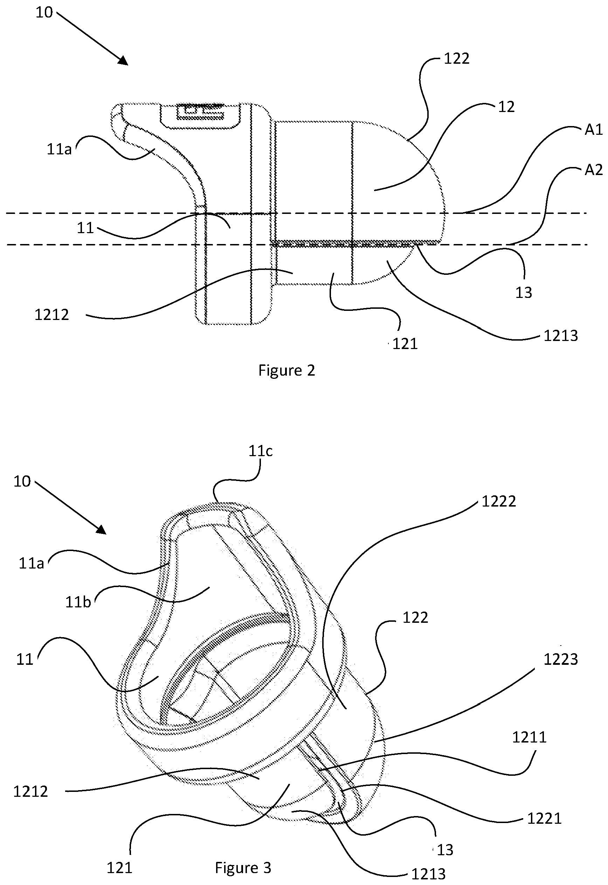

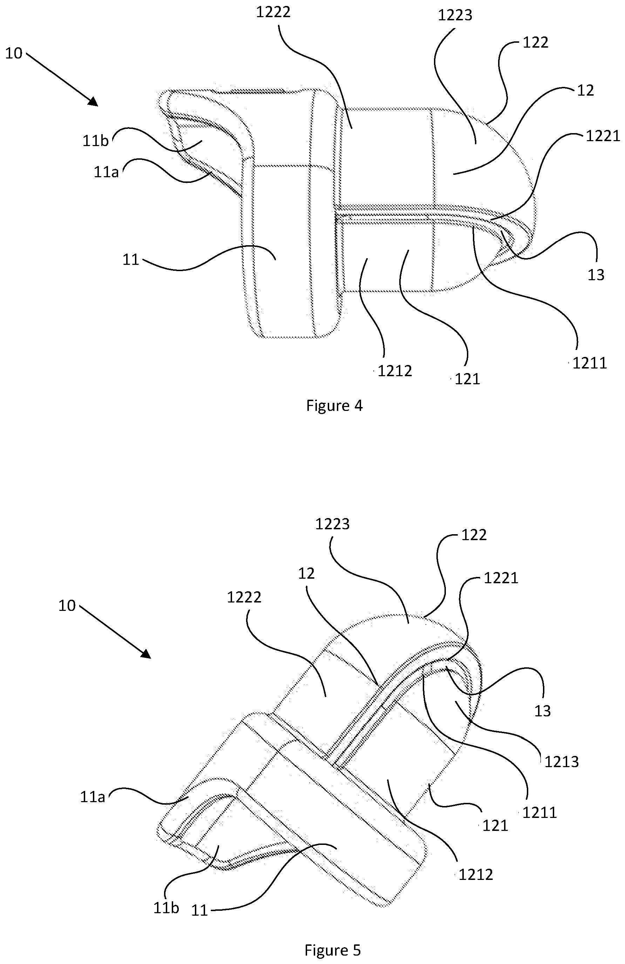

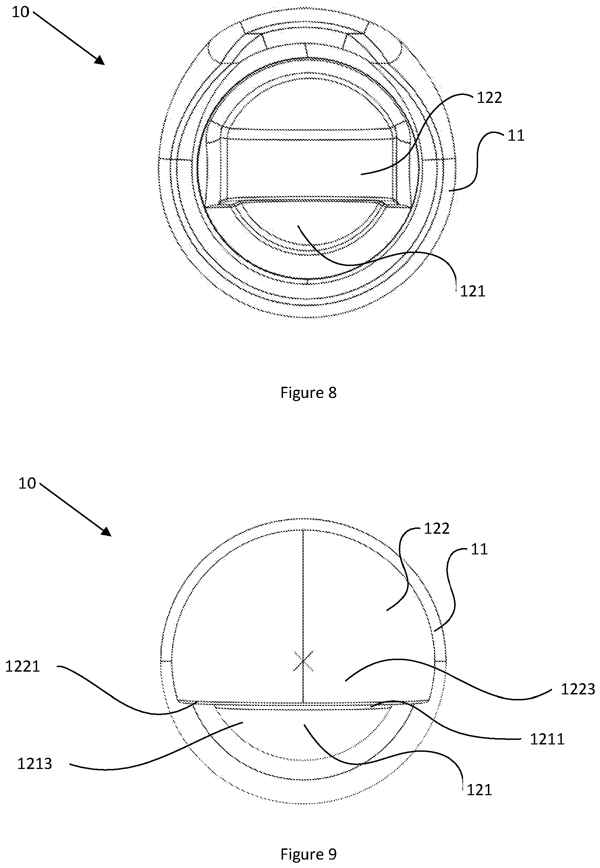

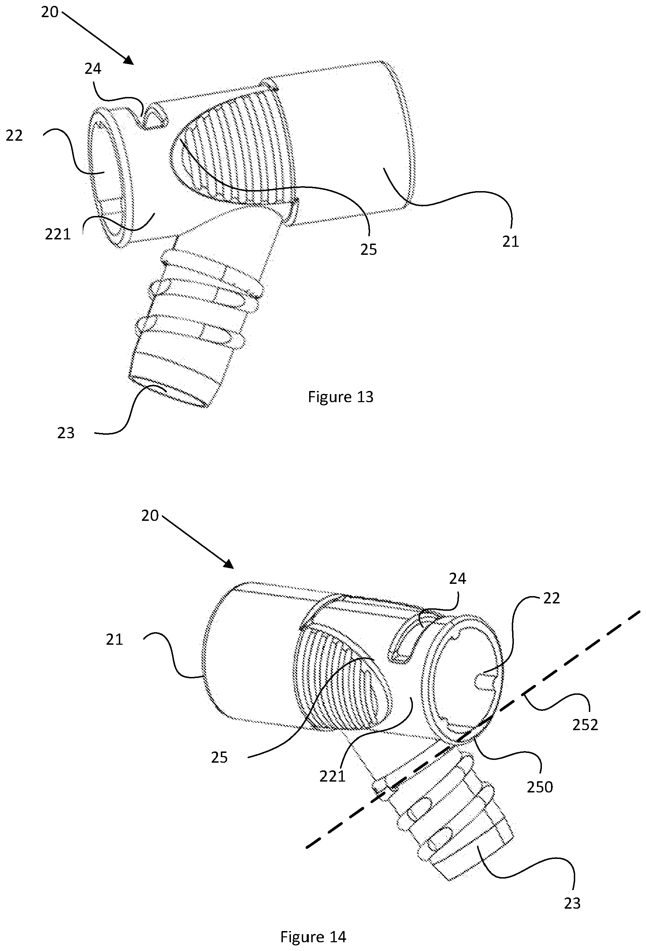

Figures 2 to 10c illustrate various views of a tracheostomy guard (also referred to as "trache guard") 10 according to a first embodiment. The tracheostomy guard 10 comprises a first portion 11 arranged to connect to a tracheostomy patient interface 20. The patient interface may for example be a tracheostomy interface such as that shown with reference to Figures 13 and 14, in fluid communication with the first portion 11, in use. With "in fluid communication" is here meant that when the patient interface 20 is connected to the first portion 11, fluid from the patient interface is allowed to enter the tracheostomy guard via the first portion 11. The tracheostomy guard 10 further comprises a second portion 12 in fluid communication with the first portion. As the second portion 12 is in fluid communication with the first portion 11, any fluid within the tracheostomy guard may travel between the first portion 11 and the second portion 12, or vice versa. The second portion 12 comprises at least one first member 121 and a second member 122 forming or defining an aperture 13 in fluid communication with the ambient environment. The aperture 13 has a size, which hereinafter may also be referred to as an effective size. is variable. The size may be variable in response to a force applied to at least part of the second portion in use, as will be further elucidated below.

The force applied to at least part of the second portion may relate to an internal pressure within the second portion, a pressure difference between the interior pressure of the second portion 12 and an ambient pressure surrounding the second portion, pressure associated with the flow of fluid or sputum within the second portion, or a physical force exerted by the user, operator, or clinician on at least part of the second portion as will be further elucidated below. Further, the specific design of the first member 121 and second member 121 and the type of force applied and on which part of the second portion 12 the force is applied will affect the way the size of the aperture varies as will be further elucidated below.

The second portion 12 is connected to the first portion 11. When the patient interface 20 is connected to the tracheostomy guard 10, in use, the second portion 12 is in fluid communication with the patient interface 20. This means that fluid entering the tracheostomy guard 10 via the first portion 11 is allowed to freely enter the second portion 12. The opposite is also true, whereby fluid in the second portion 12 may freely enter the patient interface 20.

The size of the aperture 13 may also hereinafter be referred to as an effective size. The size or effective size may relate, in effect, to the area of the aperture 13 at a given point in time. The effective size is associated with and/or affects the amount of flow that is theoretically able to pass through the aperture 13 in a given time increment (assuming the aperture 13 were to remain at that particular size over that time increment).

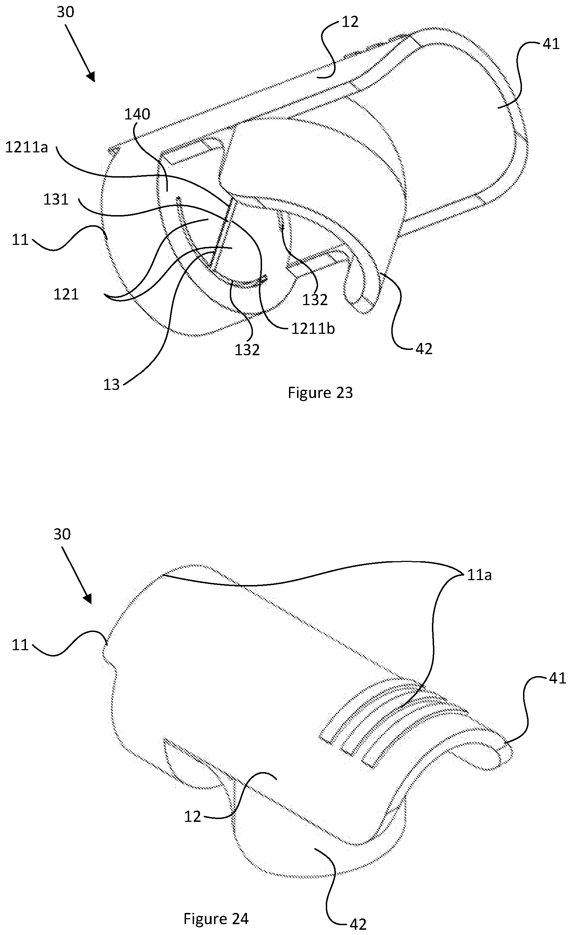

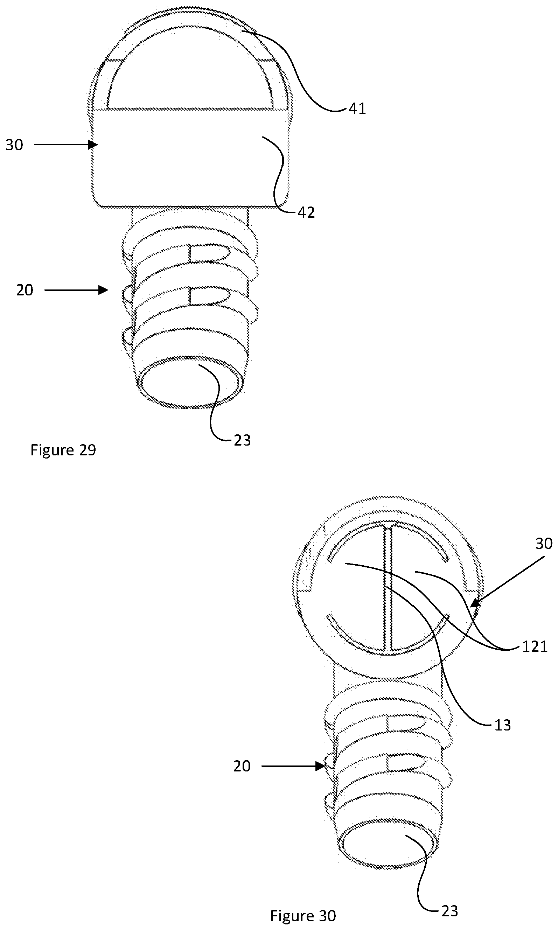

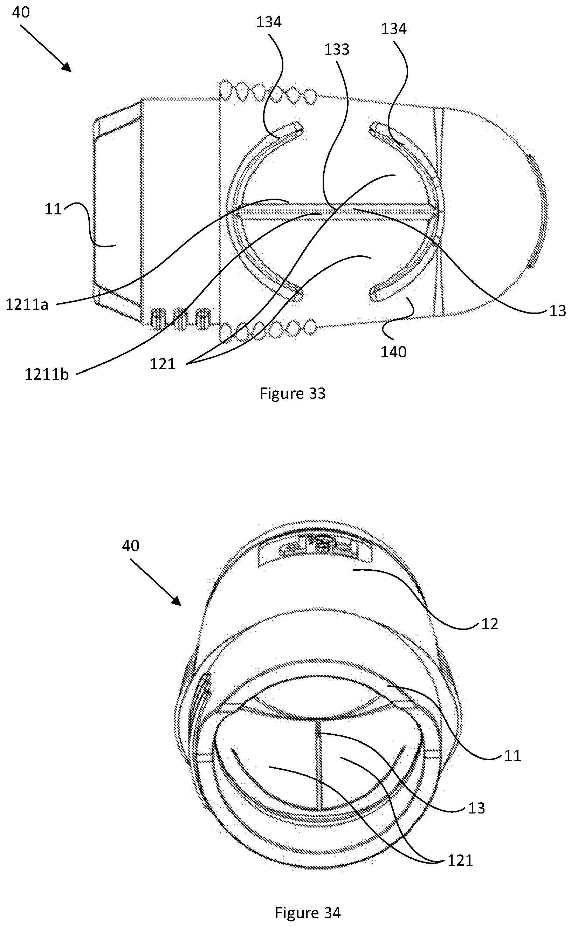

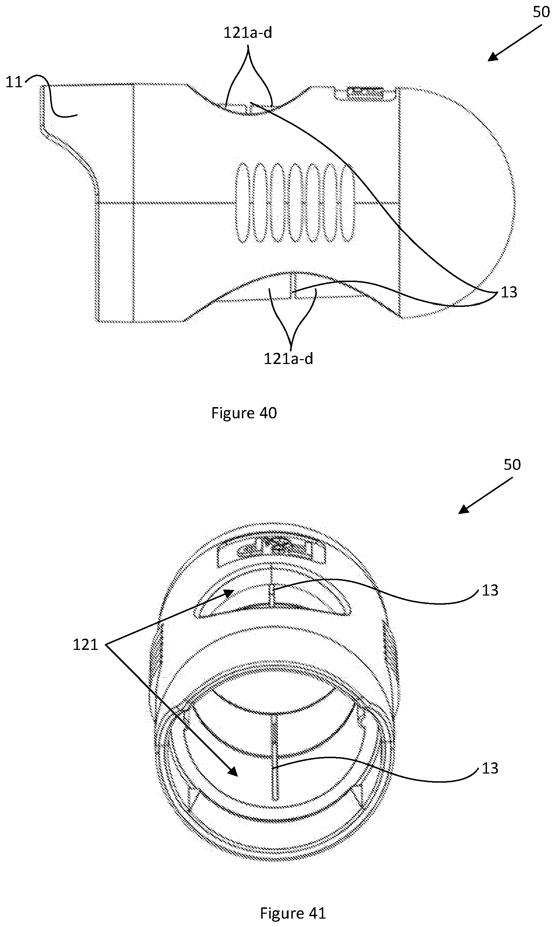

As may be observed from Figures 2 to 10c of the first embodiment the second portion 12 may comprise one first member 121 and one second member 122. However, as will be further elucidated below with reference to Figures 23 to 44, the second portion 12 may also comprise one or more first members 121.

The first member(s) 121 and second member 122 may be relatively arranged to form the at least one variable sized aperture 13.

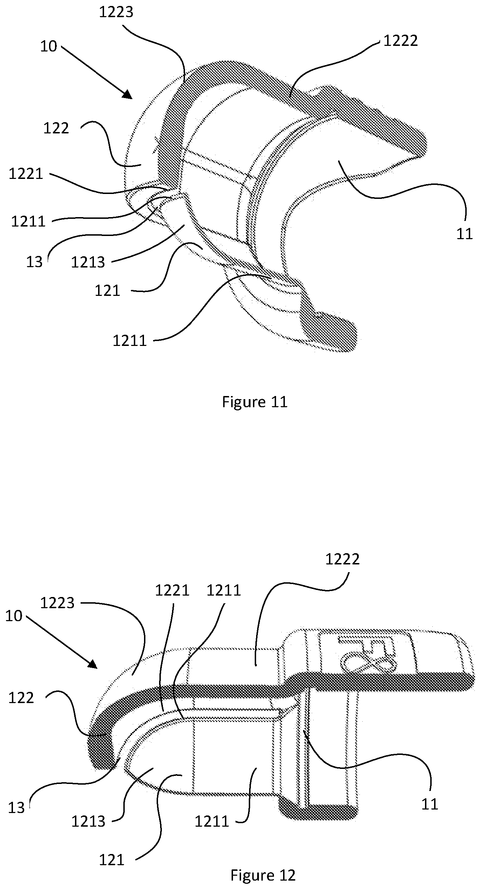

As shown with reference to Figures 2 to 12, the variable sized aperture 13 may be formed or defined between a first peripheral end 1211 of the first member(s) 121 and a second peripheral end 1221 of the second member 122. The respective peripheral ends 1211, 1221 may form respective boundary surfaces of the first member 121 and second member 122.

The first member(s) 121 may be arranged to deflect or move relative the second member 122 in response to the force applied. The deflection or relative movement of the first member(s) relative the second member 122 varies the size of the variable sized aperture 13.

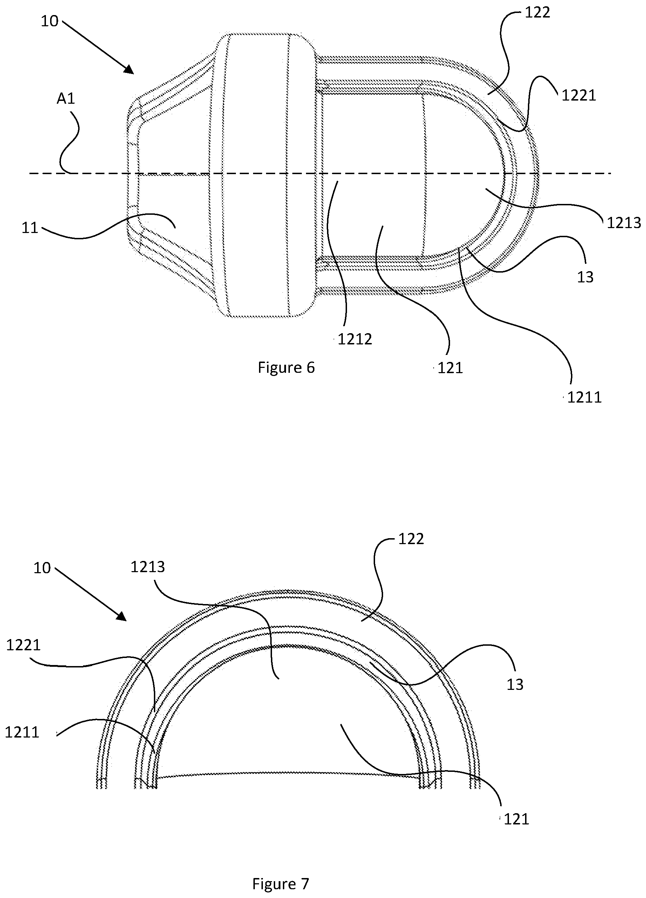

In addition, in some configurations at least part of the variable sized aperture 13 may be formed between a region of the first or second portion and the respective first member and/or second members. For example, with reference to Figure 6, if the first member 121 is not directly attached to the second member 122, at least part of the variable sized aperture may be defined between a region of the first portion or second portion and the respective members 121, 122. For example, the leftmost ends of the aperture 13 of Figure 6 may be said to be defined by a region of the first portion 11 and the respective members 121, 122.

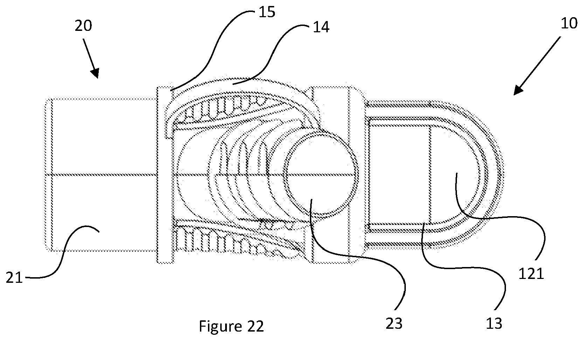

In one embodiment, the first member(s) 121 may be cantilevered off the first portion 11. Such a configuration is shown with reference to Figures 2 to 12, 15 to 30.

Additionally, or alternatively, the second member 122 may be cantilevered off the first portion 11.

In an alternative embodiment the first member 121 may be cantilevered off the second member 122. Additionally, or alternatively, the second member 122 may be cantilevered off the first member 121.

In an alternative embodiment, the first member(s) 121 may be attached to the first portion 11 by a hinging arrangement (not shown), for example a living hinge. The size of the variable sized aperture 13 varies in response to the hinged orientation of the first member(s) 121. The first member(s) 121 and the hinging arrangement may be arranged so that in a resting state, toward which the first member(s) 121 are biased to return, the variable sized aperture 13 attains its smallest size.

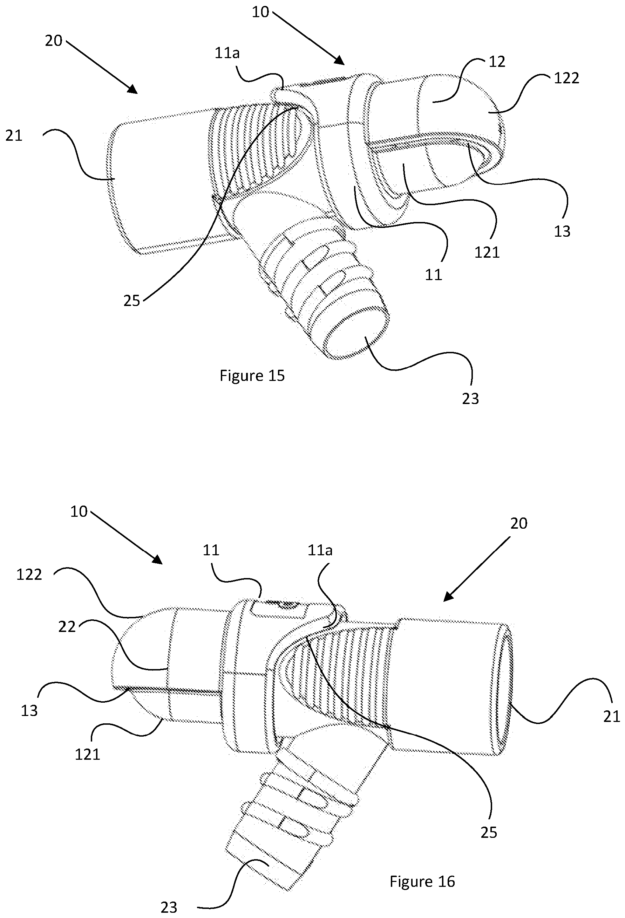

Figures 15 to 17 illustrate perspective views of the tracheostomy guard of Figures 2 to 12 connected to the patient interface of Figures 13 to 14.

The first portion 11 may have a shape adapted to couple with the patient interface 20. For example, the first portion 11 may have a circular, oval, elliptical, hexagonal, octagonal, or square cross-section. The first portion 11 may comprise a female end for mating with a male end of the patient interface 20 having a corresponding shape to that of the female end, or vice versa.

When connected to the patient interface 20 the first portion 11 is arranged to enable fluid flow between the tracheostomy guard 10 and the patient interface 20. This allows for gases flow and/or sputum to enter the tracheostomy guard 10 from the patient interface 20 via the first portion 11 and exit the tracheostomy guard 10 via the variable sized aperture 13. The second portion 12 together with the first portion 11 forms a cavity of the tracheostomy guard 10, capable of retaining an amount of sputum captured in the tracheostomy guard 10. This means that the tracheostomy guard 10 may hold a certain amount of the sputum before being released to the ambient environment via the variable sized aperture 13.

It should be appreciated that the patient interface 20, depending on the configuration, may be connected at different depths or distances into the first portion 11. In some configurations the patient interface 20 may be arranged to be connected into the first portion 11. In alternative configurations the patient interface 20 may be arranged to be connected over and/or around the first portion. Upon connection, the patient interface 20 will seal against the first portion 11.

When the patient interface 20 is connected into the first portion 11 the terminating end portion of the patient interface may in some configurations be positioned within the first portion 11. However, in other configurations the terminating end portion of the patient interface 20 may be positioned within the second portion 12. The associated penetration depth of the patient interface 20 into the first portion 11 depends on the specific design of the patient interface 20 as well as the design of the first portion 11. Hence, depending on the actual design fluid exiting or entering the patient interface 20 when connected to the first portion 11, may thus flow through a cavity formed by the first portion 11, a cavity formed by the second portion 12, or the cavity shared by the first portion 11 and the second portion 12.

The cavity formed by the second portion 12 is designed to be capable of retaining an amount of sputum captured in the tracheostomy guard 10. Throughout the embodiments disclosed here the cavity of the second portion 12 is in fluid communication with the patient interface 20 when connected to the first portion 11.

The first member 121 and/or second member 122 may comprise one or more flaps or panels. The first member(s) 121 and/or second member 122, or the flaps or panels thereof, may be configured for bi-directional movement, thus allowing for both inwards and outwards relative movement or deflection in response to the force applied to at least part of the second portion 12, in use.

The first member 121 and/or second member 122 may be physically arranged such that size of the variable sized aperture 13 is greater than zero throughout the bi-directional movement caused by the applied force to at least part of the second portion 12. The size being greater than zero means that variable sized aperture is always open allowing gases flow and sputum release therethrough.

Further, the size being greater than zero means that variable sized aperture 13 is always open. It should be appreciated that sputum may initially be retained by the second portion 12, and may be subsequently released in a delayed / controlled fashion when reaching a certain extent or amount inside the second portion 12.

The first member 121 and/or the second member 122 may be arranged so that their peripheral ends 1211, 1221 do not engage each other or other portions of the tracheostomy guard 10 in response to the force applied to at least part of the second portion, in use.

By arranging the first member 121 and second member 122 such that a distance exists therebetween for all relative positions of the first member 121 and second member 122 respectively, the variable sized aperture 13 formed or defined therebetween will remain open and gases will be allowed to flow through the variable sized aperture 13 regardless of the magnitude or extent of the applied force, in use. Since the peripheral end 1211 of the first member 121 and the peripheral end 1221 of the second member 122 do not engage each other or other portions of the tracheostomy guard 10, the variable sized aperture 13 forms a non-obstructed passageway allowing gases to flow therethrough irrespective of the magnitude of the force applied to at least part of the second portion. The first member(s) 121 and/or second member 122 are thus arranged to not completely obstruct gases from flowing inward or outward through the tracheostomy guard 10. This is advantageous in terms of patient safety as in the event of the flow generator ceasing to function the patient may draw a breath through, such as directly through, the aperture 13. In addition, the aperture 13 remaining open at all times, and the first and / or second member and / or their respective flaps being bidirectional further allows for such a technical effect.

In addition, as noted above, in high-flow therapy a substantial proportion of the air supplied to the connector vents directly to the room without being breathed by the patient and accordingly the aperture should be open, or able to become open, at all times. At the same time, however, it is desirable to provide PEEP, which requires resistance during the expiratory phase. The tracheostomy guard 10 disclosed with reference to Figures 2 to 12 may satisfy both these requirements: the variable-size aperture 13, biased towards a closed, or relatively or substantially closed, position, may provide resistance to expiratory airflow and thus facilitate the provision of PEEP, while at the same time the design of the aperture 13 may also ensure a path for escaping air is always open. These two aspects may also have the further synergistic effect that any blockage of the aperture 13 may immediately cause pressure to rise within the second portion 12, thus in turn causing the aperture 13 to be urged open.

In some embodiments, the first member(s) 121 may be arranged to move or deflect 1 to 3 mm under normal operating conditions, but larger deflections may occur when expelling sputum or secretions.

As shown with reference to Figures 2 to 12, the first member 121 may be shaped to comprise a partly tubular portion 1212 extending longitudinally from the first portion 11. Further, the first member 121 may further comprise a partly hemispherical terminating portion 1213 extending longitudinally from the partly tubular portion 1212 distal from the first portion 11. The peripheral end 1211 of the first member 121 may be said to extend along the boundary of the partly tubular portion 1212 and the partly hemispherical terminating portion 1213. Such a shape may provide the first member 121 with desired deformation properties.

By providing the first member 121 with a partly tubular portion 1212 and partly hemispherical terminating portion 1213, the extent of deflection and movement of the first member 121 may be more easily controlled. This may be enabled by providing the first member 121 with desired deformation properties, such as a degree of rigidity to prevent premature and / or excessive deflection of the first member 121, but also the required degree of flexibility to ensure or allow the first member 121 deflects in a controlled manner in response to applied force. Further, this configuration may further reduce or mitigate the effects of aeroelastic fluttering of the first member 121.

By arranging the first member 121 with a thickness being less than that of the second member 122, the first member 121 will deflect or move to a greater extent than the second member 122 in response to the force applied to at least part of the second portion 12, in use.

However, other configurations are within the scope of the invention. For instance, the desired deflection behaviour of the first member 121 relative to the second member may be achieved by their relative proportions (such as by the first member 121 having a "shallower" cross-section than the second member 122), even if they are of the same thickness. It is even within the scope of the invention for the first and second member 121, 122 to be substantially identical, wherein both are configured and dimensioned to deflect in response to applied forces so as to vary the effective size of the aperture 13, and wherein deflection of the first member 121 is additionally provided by gravity as sputum accumulates in or on the first member 121.

Further, providing the first member 122 with a partly tubular portion and partly hemispherical terminating portion, the extent of deflection and movement of the first member may be easier controlled. Also, such a shape may reduce or mitigate the effects of aeroelastic fluttering of the first member 121.

Similar to the first member(s) 121, the second member 122 may be shaped to comprise a partly tubular portion 1222 extending longitudinally from the first portion 11. Further, the second member 122 may further comprise a partly hemispherical terminating portion 1223 extending longitudinally from the partly tubular portion 1222 distal from the first portion 11. The peripheral end 1221 of the second member 122 may be said to extend along the boundary of the partly tubular portion 1222 and the partly hemispherical terminating portion 1223.

The partly tubular portion 1212 of the first member 121 and the partly tubular portion 1222 of the second member 122 may be arranged to form an essentially cylindrical cavity therebetween within the tracheostomy guard 10. The partly hemispherical terminating portion 1213 of the first member 121 and the partly hemispherical terminating portion 1223 of the second member 122 may be arranged to form an essentially half spherical cavity therebetween within the tracheostomy guard 10.

Accordingly, the cavity formed or defined between the first member 121 and the second member 122 may comprise an essentially cylindrical portion and a terminating half spherical portion.

As may be observed from Figures 2 to 12, the partly hemispherical terminating portion 1223 of the second member 122 may extend longitudinally beyond the corresponding partly hemispherical terminating portion 1213 of the first member 121. This configuration may act to deflect or direct gases or sputum towards the variable sized aperture 13 formed or defined between the first member 121 and the second member 122.

The configuration of Figures 2 to 12, with the second member 122 overlapping and projecting beyond the first member 121, may also be conducive to the aperture 13 always being open, even in the absence of applied forces, since the first member 121 is smaller than the second member 122 with the gap between them forming the aperture 13. Furthermore, since the aperture 13 generally faces downwardly in this configuration, explosively expelled sputum will tend to be arrested and redirected in a controlled manner by the partly hemispherical terminating portion 1223 of the second member 122, rather than exiting immediately and forcefully through the aperture 13. Further, when the second member 122 is arranged above the first member 121, in the "in use" configuration, gravity may act to assist in releasing deflected sputum via the partly hemispherical terminating portion 1223 of the second member 122.

In an embodiment, the first member(s) 121 may be arranged to not deflect or deform owing solely to gravity and its own weight. For example, the aforementioned shape of the first member 121, including the partly hemispherical terminating portion 1213 is designed to not deflect or deform owing solely to gravity and the weight of the first member 121. To this end, a non-planar arrangement of the first member(s) 121 may be advantageous to achieve this effect.

It should be appreciated that other shapes of the second portion, first member, and second member are also possible, such as those shown with reference to Figures 23 to 44, or any other suitable shape.

Accordingly, while some of the tracheostomy guards disclosed herein have substantially convex hemispherical terminating portions 1213, 1223, it should be appreciated that this may alternatively be substantially planar or possess a concave curvature with reference to a given reference point within the tracheostomy guard.

The variable sized aperture 13 may be formed or defined at least in part by the peripheral end 1211 of the first member 121 and the peripheral end 1221 of the second member 122. Each respective peripheral end may thus act as an aperture boundary or wall. Additionally, one or more parts of the first portion 11 or second portion 12 may act as an aperture boundary or wall.

Accordingly, the first member 121 and the second member 122 may have corresponding shapes, such as mutually complementary shapes, that form the variable sized aperture 13 therebetween.

The size of the variable sized aperture 13 may relate to a cross-sectional dimension of the aperture 13.

Additionally, or alternatively, the size of the variable sized aperture 13 may relate to an area formed or defined between the peripheral end 1211 of the first member 121, and/or the peripheral end 1221 of the second member 122, and/or optionally the part of the first portion 11 forming or defining a boundary wall of the aperture 13. The area may be defined as the non-obstructed area or passageway allowing gases to flow and optionally sputum or secretions to be released therethrough.

With reference to Figures 2 to 12, and 15 to 17 the variable sized aperture 13 may be said to be formed in a union plane (labelled A2 in Figure 2) offset by a perpendicular distance from a mid-plane comprising the longitudinal centre axis (labelled A1 in Figure 2) of the tracheostomy guard 10. As may be observed from Figure 2 the mid plane A1 extends through the first portion 11 and the second portion 12. With reference to Figure 2 the union plane may be arranged below such a mid-plane.

However, it should be appreciated that the union plane may be offset by a different perpendicular distance, which in turn affects the stiffness of the first member 121 and/or second member 122. A greater offset, i.e. a larger perpendicular distance from the midplane/longitudinal centre axis may lead to a more compliant first member 121 whereas a smaller offset may provide for a stiffer first member 121.

This is because, assuming the outer dimensions of the second portion being fixed, a greater offset, i.e. a larger perpendicular distance from the midplane/longitudinal centre axis will result in a relatively "shallower" cross-section of the first member 121 which may lead to a more compliant first member 121. On the other hand, a smaller offset resulting in a relatively "deeper" cross-section of the first member 121 may provide for a stiffer first member 121.

With reference to Figures 2 to 12, the second portion 12 comprising the first 121 and second 122 member may extend longitudinally away from the first portion 11, with reference to a longitudinal axis of the first portion 11. As may be observed in Figure 2 the second portion 12 extends away from the first portion 11 toward the right-hand side in this way. This means that both the first 121 and second member 122 may extend away, such as longitudinally away, from the first portion 11. As may be observed from Figures 2 to 6, and 10a to 10c, the second member 122 may extend beyond, such as longitudinally beyond, the first member 121 and given the partly hemispherical peripheral terminating portion 1223, the second member 122 may thus deflect gases flow and/or sputum towards the aperture 13.

This is advantageous when the second member 122 is arranged above the first member 121 with reference to the intended orientation of the tracheostomy guard 10, in use. In this configuration the variable sized aperture 13 is in fluid communication with the ambient environment below the tracheostomy guard 10, in use. As will be further elucidated below, in this configuration, gravity may assist the first member 121 to deflect outward (i.e. downward with reference to the intended use orientation of the tracheostomy guard) so as to allow for improved release of sputum contained in the second portion 12.

In some embodiments, the first portion 11 is integrally formed with the second portion 12. As such, part of the first member 121 may be integrally formed with the first portion 11, and additionally or alternatively part of the second member 122 may be integrally formed with the first portion 11.

When integrally formed the thickness of the first member 121 may be arranged to differ from the second member 122. A thinner member will deflect or move more easily in response to the force applied to at least part of the second portion 12, in use. In the embodiment shown with reference to Figures 2 to 12, the first member 121 has a thickness smaller than that of the second member 122. This means that the first member 121 will deflect or move to a greater extent than the second member 122 in response to any given force applied to at least part of the second portion 12, in use.

As will be further elucidated below the force applied to at least part of the second portion may be caused by a pressure within the second portion 12, in use.

While some of the Figures disclosed herein show the tracheostomy guard in unitary construction it should be appreciated that the tracheostomy guard according to some embodiments may instead be subdivided into a plurality of components made from different materials.

Accordingly, it should be appreciated that the first portion 11 and second portion 12 need not be formed integrally. In some embodiments the second portion 12 is releasably attached to the first portion 11. The second portion 12 may be additionally or alternatively sealingly attached to the first portion 11.

With reference to the tracheostomy guard 10 of Figures 2 to 12 and 15 to 17b, the cavity formed or defined by the first portion 11 and second portion 12, or alternatively the second portion 12 per se, is in fluid communication with the ambient environment only via the variable sized aperture(s) 13, in use. While the first portion 11 allows fluid access to the cavity via the patient interface 20, in use, the environment within the patient interface 20 does not relate to that of the ambient environment.

The tracheostomy guard 10 of Figures 2 to 12, and 15 to 17b may be said to have two access points to its cavity therein, one access point formed by the first portion 11 in fluid communication with the patient interface 20 (i.e. non ambient environment) and one access point formed by the aperture 13 in fluid communication with the ambient environment.

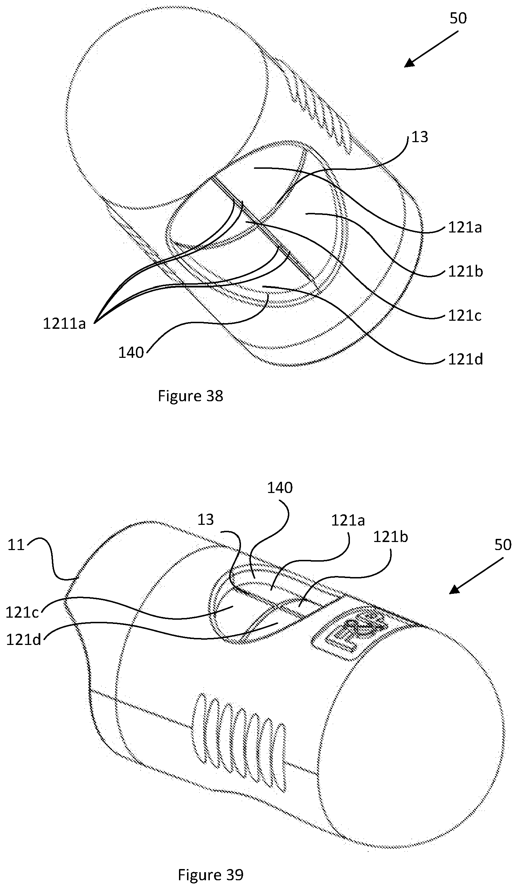

As will be further elucidated below, the tracheostomy guard 10 may be provided with more than one variable sized apertures 13 (as shown with reference to Figures 38 to 44) each being in fluid communication with the ambient environment.

It should also be appreciated that one or more constant sized, i.e. non-variable sized, apertures in fluid communication with the ambient environment may be used in combination with the variable sized aperture according to some embodiments.

When undergoing invasive or high flow ventilation there is a risk that patients may attempt to remove or adjust the patient interface 20 due to a perceived discomfort. Therefore, the at least one variable sized aperture 13 of the tracheostomy guard 10 may be positioned and/or shaped to protect against such interference. For example, by arranging the variable sized aperture 13 at a bottom side or underside of the tracheostomy guard 10 so as to be oriented downwards, in use, patient interference may be reduced and allow excess sputum to be removed or drained by gravity. Alternatively, or additionally, by arranging the variable sized aperture 13 at a top side (in relation to the orientation of the tracheostomy guard 10 in use) of the tracheostomy guard 10 patient interference may be reduced.

The first member 121 may have a first material or structural characteristic and the second member 122 may have a second material or structural characteristic. These first and second material or structural characteristics are selected to allow the size of the variable sized aperture 13 to change in response to the applied force.

According to some embodiments, the first material or structural characteristic and second material or structural characteristic relates to at least one of the following: rigidity, stiffness, flexibility, modulus of elasticity, second moment of area, length, width, and thickness.

The first and second material or structural characteristic may be provided by, or partly provided by, the profile and / or cross-section and / or dimensions of the first and second member 121, 122, respectively. For instance, as discussed above, in the embodiment of Figures 2 to 12, for a given material and a given thickness a first member 121 having a relatively "shallow" cross-section will be more prone to deflection than a first member 121 having a relatively "deep" cross-section.

In some embodiments, the second member 122 may be more rigid than the first member 121. Additionally, or alternatively, the second member 122 may be substantially stiff to prevent near total occlusion of expiratory flow path by external object(s), such as the hand of the user, impacting an exterior surface of the second member 122. Additionally, or alternatively, the second member 122 may be substantially stiff to redirect sputum which is propelled from the tracheostomy cannula and impacts an interior surface of the second member 122.

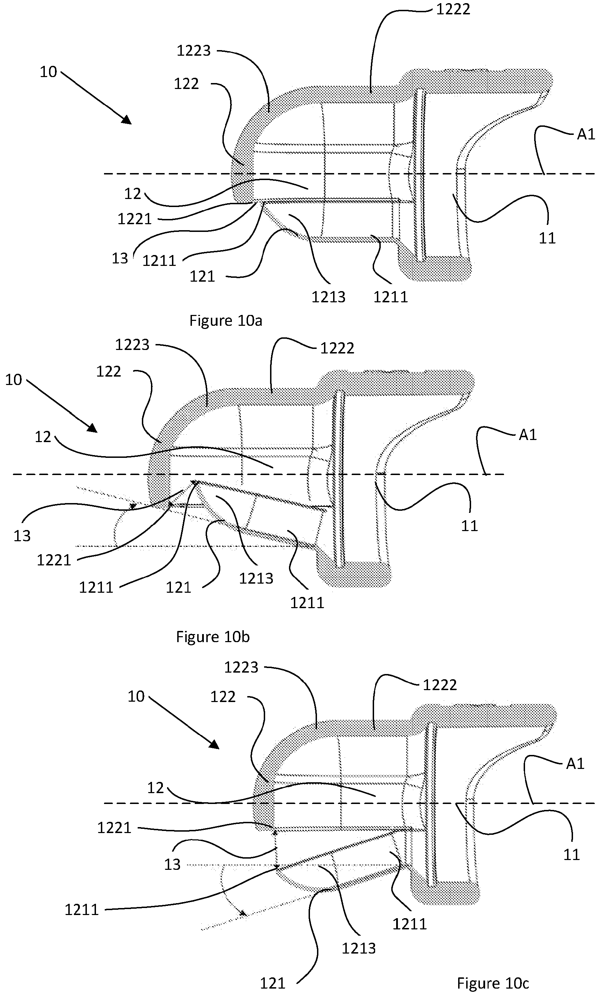

As mentioned above, the first material or structural characteristic and second material or structural characteristic may relate to a thickness or second moment of area of the first member 121 or second member 122. For example, it may be observed from Figures 10a to 10c that the first member 121 has a thickness being smaller than (i.e. less than) that of the second member 122 and this in turn, given the way the first member 121 is arranged or attached to the first portion 11, allows the first member 121 to reversibly deflect inward or outward, from its resting state shown in Figure 10a relative the second member 122. Increased thickness may lead to increased rigidity, and vice versa, so by just making the second member 122 thicker than the first member 121 the first member 121 will deflect or move to a greater extent than the second member 122 in response to any given force applied to at least part of the second portion 12, in use.

In some embodiments, the tracheostomy guard 10, the first portion 11, the second portion 12, first member 121 and/or the second member 122 may be made from a polymer, either thermoset or thermoplastic. In some embodiments the polymer is an elastomer. In some embodiments the polymer is a thermoplastic elastomer. It should be appreciated that one or more components of the tracheostomy guard 10 may be made of different materials.

In some embodiments, the tracheostomy guard 10 is made of a single material.

The first material or structural characteristic and/or second material or structural characteristic may be selected to enable reversible deformation of the first member 121 and/or second member 122 based on the force applied to at least part of the second portion 12, in use. The size of the variable sized aperture 13 may be variable based on or in response to the reversible deformation. The reversible deformation may be referred to as an elastic deformation, where the associated material returns to its original resting state shape after the associated load is applied force has been removed.

Hence, by reversible deformation is meant that the associated material will return to its resting state once the applied force causing the deformation is removed. In general, elastomeric materials allow for reversible deformation.

The reversible deformation may in some embodiments be associated with the material being deflected or bent or flexed or otherwise deformed from its resting state.

The material or structural characteristics of the first member 121 may be selected such that the first member 121 is biased to close or return to its resting state, as that shown in Figure 10a owing to the elastomeric nature of material. Additionally, or alternatively, additional structural features such as ribs or stiffening features (including increasing thickness) could be used to further enhance the behaviour or tendency of the first member 121, to close or move back to its resting state upon removal of the force applied to at least part of the second portion 12, in use.

In some embodiments, the first member 121 is sufficiently flexible to allow access for suctioning.

The tracheostomy guard 10 disclosed herein has been particularly designed to be suitable for high flow tracheostomy. In high flow tracheostomy the flow rates typically vary from about 1l/min to 150l/min depending on the type of patient. For example, high flow rates for neonatal or paediatric patients may typically be in the range of about 1l/min to about 30l/min. For most adults, high flow rates may range of about 20l/min to about 80l/min. Further, in contrast to the prior art the tracheostomy guard disclosed with reference to the various embodiments herein is provided with a variable sized aperture 13, the size of which varies in a controlled manner in response to the force applied to at least part of the second portion 12, under normal operating conditions. This means that the tracheostomy guard 10 allows for maintaining an advantageously high PEEP even for high flow applications, while still allowing for the release of secretions and sputum.

Hence, the variable size of the aperture 13 is according to some embodiments configured to allow for sputum release therethrough in response to the force applied, in use. This reduces the risk of occlusion or blockage of the patient interface 20. Blockage may adversely cause both barotrauma and/or build-up of CO2 and may further cause rebreathing of CO2. The variable sized aperture 13 disclosed herein allows for a path, such as an escape path for expired gases from the patient. To this end it should be appreciated that while a prior art fixed sized aperture (or PEEP valve arrangement) may provide for an increased PEEP at higher flow rates, such a configuration is more prone to blockage due to sputum or secretion. The deflection of the reversibly deformable first member(s) 121 and/or second member 122 disclosed herein allows for varying the size of the variable sized aperture 13, thereby reducing the risk of blockage.

Further, with the variable sized aperture 13, the tracheostomy guard 10 according to some embodiments may provide a desired PEEP in the given range of high gases flow rates, while allowing for sputum or secretions release.

As a non-limiting example, the tracheostomy guard according to some embodiments may in use allow for attaining a PEEP of:
- 1 to 3cmH₂0 at a flow rate of 20l/min
- 2 to 3.2cmH₂0 at a flow rate of 30l/min,
- 2.5 to 3.6cmH₂0 at a flow rate of 40l/min,
- 3.3 to 4.5cmH₂0 at a flow rate of 50l/min,
- 3.8 to 5.2cmH₂0 at a flow rate of 60l/min, or
- 4.5 to 6cmH₂0 at a flow rate of 70l/min.

According to some embodiments, the variable size of the aperture 13 is configured to achieve a set minimum positive end expiratory pressure (PEEP), in use. The set minimum PEEP may be selected from a range of: 1cmH₂0 to 7cmH₂0. As seen above, the PEEP achieved depends on the flow rate and selection of the set minimum PEEP is at the discretion of the clinician.

It should be appreciated that for any given gases flow rate decreasing the size of the variable sized aperture 13 will in general increase the PEEP, and vice versa.

Depending on the extent of the applied force experienced by at least part of the second portion 12, in use, the relative orientation between the first member 121 and second member 122 will change, leading to the aperture 13 formed or defined between the first member 121 and the second member 122 changing or varying in size.

The force applied to at least part of the second portion 12, in use, which in turn varies the size of the variable sized aperture 13, by varying the relative orientation of the first member 121 and second member 122 may relate to, be associated with, or at least partly result from an internal pressure of or within said second portion 12.

The respective configuration, shape, material characteristics, and/or structural characteristics of the first member 121 and second member 122 affects how the first member 121 and/or second member 122 will behave in terms of movement and/or deflection upon an applied force. This in turn affects how the parts of the first member 121 and second member 122 will behave when experiencing the applied force. Further, the part of the second portion, such as at least a part of the first member 121 and/or second member 122, at which the force is applied will affect how the first member 121 and second member 122 will behave when experiencing the applied force.

As an example, when taking structural characteristics in isolation, if the first member 121 and second member 122 are both made of the same material, and the first member 121 is made thinner than the second member 122, for a given configuration the first member 121 will tend to move or deflect to a larger extent from its original orientation than the second member 122 would when experiencing the same applied force. This scenario may be observed in Figures 10a to 10c where the first member 121 is made thinner than the second member 122.

As further elucidated herein, the configuration and/or shape of the respective member 121, 122 also affects the behaviour in terms of movement and/or deflection of that member 121, 122 when experiencing an applied force.

In at least one example, the second member 122 is designed to provide for lesser or substantially lesser deflection than the first member 121 when subject to a given applied force. Turning back to Figures 10a to 10c, the second member 122 is designed to provide for a substantially lesser deflection when experiencing an applied force than the first member 121, when subject to a given applied force.

The internal pressure of the second portion 12 may at least in part relate to the internal gases pressure within the cavity formed by the first portion 11 and the second portion 12. Additionally, or alternatively, the internal pressure may at least in part relate to the resulting pressure formed by the weight of sputum or secretions acting on the second portion 12, e.g. the first member 121 thereof, in use. The force applied to at least part of the second portion, such as the force applied to the second portion 12 or at least part of same, may at least in part relate to the weight of sputum or secretions present within the second portion 12, in use.

The force applied to at least part of the second portion 12, in use may further be associated with a flow rate of gases in the tracheostomy guard 10. For example, when the patient coughs the increased gas flow rate through the tracheostomy guard 10 will result in an increased force applied to at least part of the second portion 12, such as the first member 121 and/or second member 122 thereof, whereby the size of the aperture 13 varies accordingly.

In use, the force applied to at least part of the second portion 12 may according to some embodiments be referred to as an operational force that is dependent *inter alia* on the supplied gases flow rate, the patient's breathing cycle, abrupt pressure changes due to the patient coughing and/or the amount of sputum or secretions present in the tracheostomy guard 10.

Additionally or alternatively, the force applied to at least part of the second portion 12 may further relate to a physical force, e.g. a force exerted by the user, such as a clinician, to deform the first member(s) 121 and/or second member 122 to vary the size of the variable sized aperture 13. Optionally, a physical force may be applied by means of a tool, such as a suction catheter, inserted through the aperture 13, in use.

In some embodiments the force applied, i.e. the force applied to at least part of the second portion, relates to a resultant or net force of all forces acting on the second portion 12 or associated parts thereof, including the first member 121 and/or second member 122 thereof.

In some embodiments, the size of the variable sized aperture 13 changes when a positive and/or negative resultant force is applied to the second portion 12 or a part thereof, including the first member 121 and/or second member 122 thereof. In this way, the size of the variable sized aperture 13 changes as a consequence of a positive and/or negative resultant force being applied to the second portion 12 or a part thereof, including the first member 121 and/or second member 122 thereof. A resultant positive force is here defined as a force, such as a force acting on at least part of the second portion 12, having a component directed outwards from the second portion 12towards the ambient environment. A resultant negative force is here defined as a force, such as a force acting on at least part of the second portion 12, having a component directed inwards or towards the second portion 12 from the ambient environment. When the resultant force is zero the tracheostomy guard 10 may be said to be in its resting state. In other words, the resting state may be defined as the state of rest or balance where all external forces acting on the tracheostomy guard 10 are in equilibrium. For example, the tracheostomy guard 10 may be in its resting state when not connected to the patient interface 20 or when connected to the patient interface 20 but not to the flow generator, in use, whereby the interior pressure of the second portion 12 is equal to that of the ambient pressure.

In some embodiments, the resultant applied force may also be referred to as, or be related to, the relative pressure difference between the interior pressure acting on the second portion 12 and the exterior pressure acting on the second portion 12. Hence, whenever the interior pressure is higher than the exterior pressure, the applied force may be said to relate to a positive relative pressure difference, and vice versa, which in turn acts to change the size of the variable sized aperture 13.

The size of the tracheostomy guard 10 can be chosen such that it is not obstructive to a patient and/or caregiver, but provides a sufficient barrier to occlusion and/or is able to deflect patient secretions such that they are not communicated with a caregiver, or put differently expelled or projected to the environment in an uncontrolled manner.

In some embodiments, the area of the variable sized aperture 13 in the resting state is approximately 25 to 30mm², such as 27 mm².

As mentioned previously, the variable sized aperture 13 formed or defined between the first member 121 and the second member 122 will vary in size in response to a force applied to at least part of the second portion 12, in use. The force applied affects the relative position between the first member 121 and second member 122.

In some embodiments, such as with reference to Figures 10a to 10c, the first member 121 may be arranged to move in relation to the second member 122 in response to the applied force, thereby varying the size of the variable sized aperture 13.

Whether the size of the variable sized aperture 13 increases or decreases depends on the change in the force applied to at least part of the second portion 12. For example, when the force applied relates to and/or comprises a resultant positive force, defined as a force having a component directed outwards from the second portion, i.e. outwardly from the second portion, towards the ambient environment, this will cause an outward deflection or movement of the first member(s) 121 and/or second member 122.

Alternatively or additionally, the size of the variable sized aperture may also be affected by which part of the second portion the force is applied to.

When the force applied relates to and/or comprises a resultant negative force, defined as a force having a component directed inwards from the ambient environment towards the second portion 12, i.e. inwardly from the ambient environment, this will cause an inward deflection or movement of the first member(s) 121 and/or second member 122.

For all configurations except for the resting state, where the variable sized aperture 13 attains its smallest size, the size of the variable sized aperture 13 will decrease in response to resultant negative force change and increase in response to a resultant positive force change. Hence, for any given orientation except for the resting state, when there is a negative change of the resultant force the size of the variable sized aperture 13 will decrease. Here, a negative change in resultant force implies a scenario where the new resultant force is smaller or lower than the original resultant force before the change. Further, for any given orientation except for the resting state, when there is a positive change of the resultant force the size of the variable sized aperture 13 will increase. Here, a positive change in resultant force implies a scenario where the new resultant force is larger or higher than the original resultant force before the change. In other words, larger and higher in this context implies a larger magnitude of the associated resultant force.

Under some conditions (such as for a symmetrical aperture configuration attaining the same size for a negative and positive resultant force of the same magnitude or absolute value, or as long as there are no crossings of the resting state for non-symmetrical aperture configurations), the size of the variable sized aperture 13 may be said to decrease if the absolute value (i.e. magnitude) of the resultant force decreases (i.e. gets smaller), and will increase if the absolute value (i.e. magnitude) of the resultant force increases (i.e. gets larger). Under these conditions, for any given orientation except for the resting state, when there is a decrease in the absolute value (magnitude) of the resultant force the size of the variable sized aperture 13 will decrease. Further, under these conditions for any given orientation except for the resting state, when there is an increase in the absolute value (magnitude) of the resultant force the size of the variable sized aperture 13 will increase.

Figures 10a to 10c show respective cross-sectional views the tracheostomy guard 10 of the first embodiment when subject to three different forces applied to at least part of the second portion 12. The different forces applied each result in the first member 121 moving relatively to the second member 122, thereby varying the size of the variable sized aperture 13. Figure 10a illustrates a tracheostomy guard 10 when in its resting state, i.e., wherein the size of the variable sized aperture attains its smallest size. In this configuration all the net forces acting on at least part of the second portion 12 are in equilibrium. Figure 10b illustrates a situation where a resultant negative force is applied to at least part of the second portion 12. As a result of the negative change in resultant force, the first member 121 is deflected or moved inwards from its resting state position shown in Figure 10a. The Figure 10b configuration may for example occur in the unlikely event there is a complete or partial blockage in the patient interface 20, or a complete or partial blockage in the gases flow from the gases source, and the patient inhales. Figure 10c represents the configuration of the tracheostomy guard 10 when a resultant positive force is applied to at least part of the second portion 12, in use. As mentioned previously, the resultant positive force may be due to the gases flow rate supplied to the patient via the patient interface 20 from the gases source, the patient exhaling, the patient coughing, or due to the weight of sputum (not shown) acting on at least part of the second portion 12, such as the first member 121 and/or the second member 122.

If the applied force, such as the resultant applied force, reduces, when the tracheostomy guard 10 is already at a negative force side of its resting state configuration, i.e. when the first member 121 (as shown in Figure 10b) and/or second member 122 (which may not be observed from Figure 10b as the inward deflection of the second member 122 is substantially negligible in Figure 10b for that particular design) are deflected or moved inwards from the resting state the variable sized aperture 13 will increase by the tracheostomy guard 10 configuration moving further and further away from the resting state on the negative force side of the resting state.

When the tracheostomy guard is at a negative force side of its resting state configuration this means that the resultant force applied to second portion is defined as negative. In other words, all forces acting inwardly on said second portion or at least part thereof are larger than those acting outwardly on the second portion. In this configuration the at least one first member will be deflected inwards.

When the tracheostomy guard is at a positive force side of its resting state configuration this means that the resultant force applied to second portion is defined as positive. In other words, all forces acting inwardly on said second portion or at least part thereof are smaller than those acting outwardly on the second portion. In this configuration the at least one first member will be deflected outwards.

When the tracheostomy guard is already at its resting state this means that the resultant force applied to second portion is defined as zero. In the resting state, all forces acting inwardly on said second portion or at least part thereof are in equilibrium with those acting outwardly on the second portion. In this configuration the at least one first member will be deflected neither inwards nor outwards.

Depending how all of the forces acting inwardly on the second portion and all of the forces acting outwardly on the second portion vary over time, this will affect whether the tracheostomy guard will attain the negative force side, positive force side, or resting state.

For completeness, it is noted that the absolute value of the applied negative resultant force increasing could alternatively be referred to as the force "reducing", in the sense of becoming a larger negative value. However, in this description the terminology "absolute value increasing / decreasing" is used to indicate changes in the magnitude of the force *per se*; and "positive / negative" are used to indicate the general direction of said force, with "positive" indicating that the force is acting in an outward direction relative to the second portion, and "negative" indicating that the force is acting in an inward direction relative to the second portion.

On the other hand, if the applied force, such as the resultant applied force, reduces, when the tracheostomy guard 10 is already at a positive force side of its resting state configuration, i.e., when the first member 121 and/or second member 122 are deflected or moved outwards from the resting state such as in Figure 10c, the variable sized aperture 13 will decrease as the reduction in applied force will bring the configuration closer to the resting state, but after passing the resting state shown in Figure 10a, provided the applied force is still reducing, the variable sized aperture 13 will increase by the tracheostomy guard 10 configuration moving further and further away from the resting state on the negative force side of the resting state as shown in Figure 10b.

Alternatively or additionally, if the absolute value of the applied positive resultant force decreases, the variable sized aperture 13 will decrease as the reduction in the absolute value of the applied force will bring the configuration closer to the resting state (shown in Figure 10a). If, however, a negative force (i.e. inwardly acting force) is applied (or continues to be applied) after the tracheostomy guard 10 has reached the resting state, then the variable sized aperture 13 will increase by the tracheostomy guard 10 configuration moving further and further away from the resting state on the negative force side of the resting state as shown in Figure 10b.

Depending on the resultant positive or negative applied force acting on at least part of the second portion 12, the size of the variable sized aperture may eventually stabilise (i.e. reach an equilibrium state) and be maintained until the applied force is changed further. In this stabilised state the first member 121 and second member 122 will maintain their respective orientation and deflection.

If the applied force increases when the tracheostomy guard 10 is already at a positive force side of its resting state configuration (such as when subject to a positive, outward-acting, resultant applied force and the absolute value of the positive resultant applied force increases), the variable sized aperture will increase by the tracheostomy guard configuration moving further and further away from the resting state on the positive force side of the resting state.

On the other hand, if the tracheostomy guard 10 is already at a positive force side of its resting state configuration (i.e. subject to a positive (outward-acting) resultant applied force) and the absolute value of the positive resultant applied force decreases, the variable sized aperture will decrease by the tracheostomy guard configuration moving closer to the resting state.

On the other hand, if the applied force increases when the tracheostomy guard 10 is already at a negative force side of its resting state configuration (such as when subject to a negative (inward-acting) resultant applied force), as illustrated in Figure 10b, and the absolute value of the negative resultant applied force decreases), the variable sized aperture will decrease as the increase in applied force will bring the configuration closer to the resting state, as shown in Figure 10a, but after passing the resting state the variable sized aperture will increase by the tracheostomy guard configuration moving further and further away from the resting state on the positive force side of the resting state, as shown in Figure 10c.

In this way, the variable sized aperture may be said to decrease as the decrease in the magnitude of the applied force will bring the configuration closer to the resting state, as shown in Figure 10a.

On the other hand, if the tracheostomy guard 10 is already at a negative force side of its resting state configuration (i.e. subject to a negative (inward-acting) resultant applied force), as illustrated in Figure 10b, and the absolute value of the negative resultant applied force increases, the variable sized aperture will increase as the increase in the magnitude of the applied force will urge the first member 121 further inwardly and away from the resting state.

It will be understood that the second portion 12 is configured such that the aperture size may increase in a bi-directional manner; that is to say, the smallest size of the aperture is when the second portion 12 is in a resting state, and an applied force (whether in the inward (negative) or outward (positive) direction) will cause the aperture to increase in size via the first member 121 being urged away from the resting state, whether inwardly or outwardly. It will also be understood that, if the first member, having been displaced, moves back towards the resting state, and a force in the other direction is applied or continues to be applied, then the first member 121 will move past the resting state in the other direction, thus again increasing the size of the aperture. Thus, if the tracheostomy guard 10 is at the negative force side of its resting state and the absolute value (magnitude) of the negative resultant applied force is decreasing, the first member 121 will move toward the resting state, until the resultant applied force becomes or passes zero, at which point the tracheostomy guard attains its resting state. If a positive resultant applied force is then applied, the first member 121 will move outwardly away from the resting state.

As explained above, depending on the magnitude of the applied force, such as the resultant applied force, the system may reach an equilibrium state at which the size of the variable sized aperture will stabilises and is maintained until the applied force is changed further. In this stabilised state the first member 121 and second member 122 will maintain their respective orientation and deflection.

Any change of the applied force, such as a change of its associated absolute value, from the resting state configuration will lead to an increased size of the variable sized aperture. For example, if the tracheostomy guard is in its resting state and is subject to a resultant negative force the first member 121 and/or second member 122 will deflect inwards and the size of the variable sized aperture will be increased. On the other hand, if the tracheostomy guard is in its resting state and is subject to a resultant positive force the first member 121 and/or second member 122 will deflect outwards whereby size of the variable sized aperture will be increased as well.

As noted elsewhere, it will be understood that the applied resultant force may be at least partly related to, or caused by, the pressure within the second portion 12 of the tracheostomy guard 10, the ambient environment pressure outside the second portion 12 of the tracheostomy guard 10, and/or a difference in pressure between the interior of the second portion 12 and the ambient environment outside of / around the second portion 12. Alternatively, or additionally it should be appreciated that the resultant applied force may also be at least partly related to, or caused by, other forces (such as weight of sputum or a force from the user or clinician) exerted onto at least some parts of the second portion.

In an alternative embodiment, the tracheostomy guard 10 may be designed to attain the smallest size of the variable sized aperture in response to a base line gases flow rate. The base line gases flow rate may relate to the lowest high flow rate associated with the patient type, such as 20l/min for adult patients, or 1l/min for neonatal patients. Alternatively, the base line gases flow rate may relate to any high flow rate selected from the aforementioned high flow rate ranges.

It will be understood that, while the bidirectional operation has been described above with reference to Figures 10a and 10c which depict the Figure 2 embodiment of the tracheostomy guard, having a movable / deflectable first member(s) 121, in other embodiments the aperture 13 and surrounding portions of the second portion 12 of the tracheostomy guard 10 may likewise behave in a bidirectional manner in response to applied forces; and, depending on the embodiment, this may be a function of the first member 121, the second member 122, and / or another region(s) or portion(s) of the second portion 12 being movable in response to applied force.

The patient interface 20 referred to herein may be a tracheostomy interface arranged to engage a patient at a tracheostomy opening. The tracheostomy patient interface 20 may optionally be arranged with a leak to reduce the chances of a barotrauma occurring by the delivery of high flow.

Figures 13 and 14 illustrate perspective views of a patient interface 20. The patient interface 20 comprises a first end 21 and a second end 22 connected by a body. The first end 21 of the patient interface 20 is configured to fluidly connect with the airway of a patient, for example, by coupling with a tracheal tube (not shown). The second end 22 of the patient interface 20 is configured to couple with the first portion 11 of the tracheostomy guard 10. A port 23 couples with a supply tube or conduit 101 which delivers gases to the first end 21 from a gases source (not shown). The patient interface 20 may or may not comprise a vent to prevent accidental occlusion of the patient interface 20, by a patient, caregiver, or due to patient secretions within the patient interface 20.

The gases source may be any device capable of supplying a gases flow to the patient interface in use, such as a blower, flow generator, ventilator, and/or humidification apparatus.

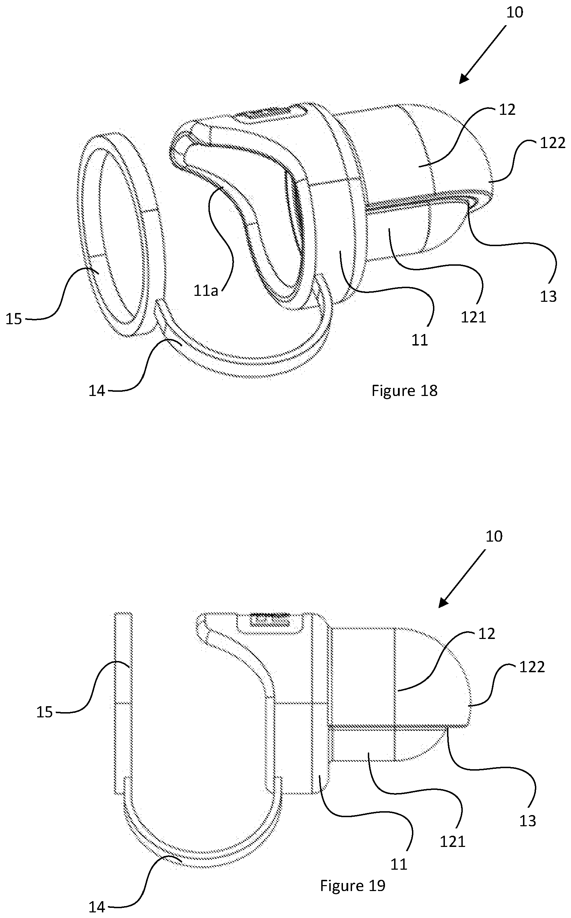

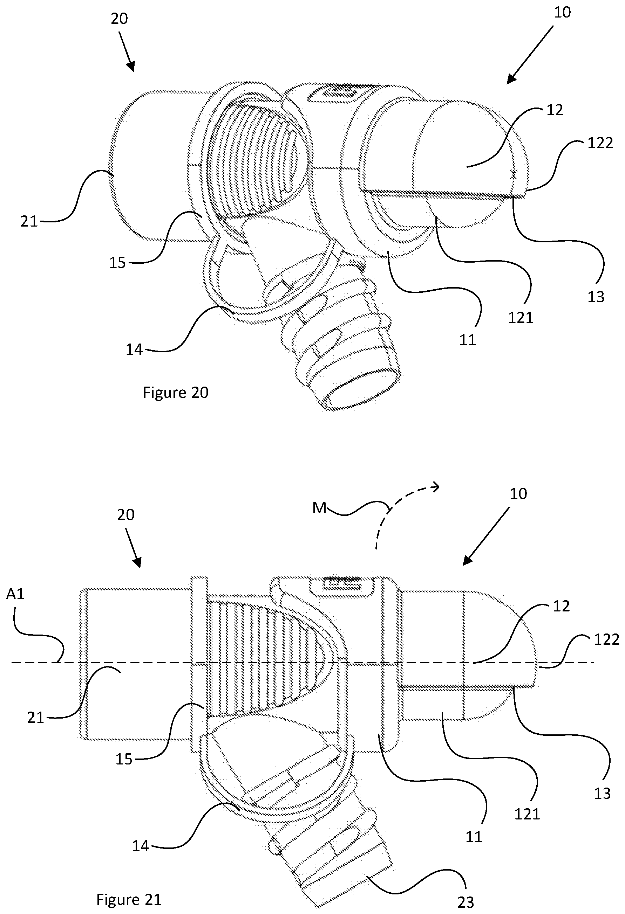

According to a second embodiment, with reference to Figures 18 to 22, the tracheostomy guard 10 further comprises a patient interface retaining unit 14, 15 to allow the tracheostomy guard 10 to be coupled to the patient interface 20. The patient interface retaining unit 14, 15 allows for the tracheostomy guard 10 to be retained to the patient interface 20 independently of the first portion 11 being connected or disconnected to the patient interface 20. Hence, the patient interface retaining unit 14, 15 prevents the tracheostomy guard 10 from becoming separated or detached from the patient interface 20, for example when the tracheostomy guard 10 is disconnected for optional cleaning, in use. The patient interface retaining unit 14, 15 may comprise a flexible member 14 (such as a tether) attached at one end of the patient interface attachment member 15, which may comprise a loop arrangement. The patient interface attachment member 15 may be arranged to connect to, or proximate to, the first end 21 of the patient interface 20 that is arranged to fluidly connect to the airway of the patient, in use, or any other suitable member of the patient interface 20. The patient interface retaining unit 14, 15 may be arranged around or proximate to the first end 21 of the patient interface 20 as shown with reference to Figures 20 to 22. The tether or flexible member 14 may be releasably or integrally attached to the first portion 11 of the tracheostomy guard 10.

Figures 20 to 22 illustrate, respectively, a perspective, side, and bottom plan views of the tracheostomy guard 10 of the second embodiment when connected to the patient interface 20 of Figures 13 and 14.

It should be appreciated that a patient interface retaining unit 14, 15 may be provided to any one of the tracheostomy guard embodiments disclosed herein.

In some embodiments, the tracheostomy guard 10 comprises a first portion 11 having an end arranged to releasably attach to a patient interface 20. The first portion 11 may be configured to sealingly attach to the patient interface 20. The first portion 11 may comprise an alignment feature 11a for alignment with a corresponding alignment feature 25 of the patient interface 20. The first portion 11 may further comprise an attachment member 11b arranged to attach to a corresponding attachment member 221 of the patient interface 20. The attachment member 11b may be arranged to mate with the attachment member 221 of the patient interface 20 when the first portion 11 is attached to the patient interface 20. The attachment member 11b may be configured to sealingly attach to the corresponding attachment member 221 of the patient interface 20. The attachment member 11b may be further arranged to disconnect from the corresponding attachment member 221 of the patient interface 20 upon the application of a directional force to the tracheostomy guard 10.

The directional force may have a component opposite that of a general mating direction when the attachment member 11b mates with the corresponding attachment member 221 of the patient interface 20.

The attachment member 11b may be arranged to disconnect from the corresponding attachment member 221 of the patient interface 20 by the application of a directional force opposite the mating direction, such as a directional force substantially opposite the mating direction, followed by the application of a moment to the tracheostomy guard 10 in relation to the patient interface 20.

In this way, the attachment member 11b may be arranged to disconnect from the corresponding attachment member 221 of the patient interface 20 by the application of a directional force substantially opposite the mating direction, for instance by imposing on the tracheostomy guard 10 a force acting away from the patient interface 20 substantially along the longitudinal centre axis of the first portion 11 (labelled A1 and shown as a dashed line in Figures 2, 6, 10a to 10c, 17a to 17b, 21, 27, 35, and 42). This may be referred to as a "push-fit / pull-release" configuration whereby the user may simply pull the tracheostomy guard 10 off the patient interface 20 in order to disconnect the two.

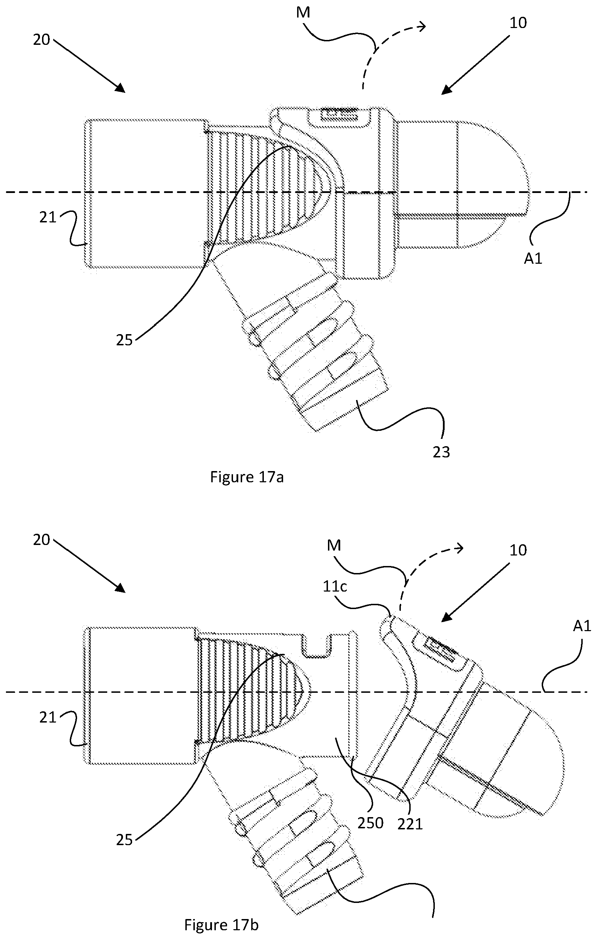

The moment may be directed around a transverse axis, or substantially transverse axis, of the first portion 11, intersecting the longitudinal centre axis of the first portion 11. The moment is indicated by the dotted arrow (labelled M) in Figures 17a, 17b, 21, 27, 35, and 42.

The directional force is directed away from a longitudinal centre axis of the first portion 11. The longitudinal centre axis (labelled A1) is shown as a dashed line in Figures 2, 6, 10a to 10c, 17a to 17b, 21, 27, 35, and 42.

For example, the directional force or moment may be imposed about a lower edge 250 of the patient interface 20. The lower edge 250 may form an effective "pivot point" of the tracheostomy guard 10 when being removed via application of the moment. The direction force or moment may be directed around an axis, for example axis 252 that passes through said lower edge 250, that is substantially transverse to and/or intersecting the longitudinal centre axis of the first portion 11.

Alternatively, attachment member 11b may be arranged to disconnect from the corresponding attachment member 221 of the patient interface 20 upon application of a moment by the patient or user.

In this way, the transverse or substantially transverse axis such as that indicated as a dotted line 252 with reference to Figure 14, intersects with the lower edge 250 of the patient interface 20, such that said lower edge 250 forms an effective "pivot point" of the tracheostomy guard 10 when being removed via application of the moment. In use, the user applies a force to the first portion (such as via lip 11c, discussed next) that generates the moment which causes the tracheostomy guard 10 pivot away from, and thus detach from, the patient interface 20.

In some embodiments, the attachment member 11b may comprise an attachment release lip, (e.g., attachment release lip 11c) for mating with a corresponding attachment member 221 of the patient interface 20.

In some examples, the attachment release lip 11c may be configured to enable a user to impose the moment required to disconnect the attachment member 11b from the corresponding attachment member 221. For instance, the release lip 11c may be formed as a tab, tongue or protrusion which the user can grip and pull to effect the moment; the pulling motion generally following the arc indicated by the dashed arrow M (up and then out). Thus, this configuration may be referred to as "pivot-release", in that, rather than applying a force substantially directly opposed to the mating force, the user instead applies a force that results in a moment about point 250, thereby causing the tracheostomy guard 10 to pivotally disengage from the patient interface 20. Further to the above, in some embodiments the attachment member 11b may comprise an attachment release lip 11c. The attachment release lip 11c may form part of the attachment member 11b configured to attach to corresponding attachment member 221 of the patient interface 20. Furthermore, the attachment release lip 11c may be configured to enable a user to impose the moment required to disconnect the attachment member 11b from the corresponding attachment member 221. For instance, the release lip 11c may be formed as a tab, tongue or protrusion which the user can grip and pull to effect the moment; the pulling motion generally following the arc indicated by the dashed arrow M (up and then out).

At least part of the attachment member 11b, such as the attachment release lip 11c, may have a tubular smooth surface to mate with a corresponding smooth surface of the corresponding attachment member 221 of the patient interface 20, for example as shown with reference to Figures 2 to 19. Such a configuration provides for a friction fit between the tracheostomy guard 10 and the patient interface 20, such as to provide a sealing connection.

The friction fit may be achieved by a friction fit mechanism formed by the attachment member 11b and the corresponding attachment member 221 of the patient interface 20. In this regard, the cross-sections of the respective attachment members (11b, 221) may be complementary, for example both may be circular or otherwise similar in shape to facilitate snug coupling therebetween, such as to provide a sealing connection.

The friction fit mechanism is configured to releasably couple the tracheostomy guard 10 and the patient interface 20, such that a user can connect and/or disconnect the tracheostomy guard 10 from the patient interface 20. This can be by way of push-fit and/or pull release, whereby the shape of the guard 10 is circular or otherwise similar in shape to the patient interface 20 so it can couple snugly thereto, such as to provide a sealing connection.

Alternatively, or additionally, the disconnection may be achieved, for instance, in the above-discussed manners, utilising pull-release or pivot-release.

A user may temporarily disconnect the tracheostomy guard 10, for example, for cleaning, or to better access the patient interface 20. The coupling mechanism or friction fit mechanism is configured to facilitate coupling between the patient interface 20 and the tracheostomy guard 10, with a sufficient retention force to reduce the likelihood of accidental disconnection or removal of the tracheostomy guard 10. The force to remove the tracheostomy guard 10 from the patient interface 20 should therefore be greater than forces that are likely to be encountered during use. Minimal force should be required to couple the tracheostomy guard 10 to the patient interface 20 in use to reduce discomfort to a patient. In some embodiments, the force required to decouple the tracheostomy guard 10 from the patient interface 20 is greater than the coupling force. The direction of the force required to detach the tracheostomy guard 10 from the patient interface 20 is shown by the dashed arrow (labelled M) in Figures 17a and 17b.

Requiring the application of a moment in order to remove the tracheostomy guard 10, such as in the "pivot-release" configuration discussed above, is one way of ensuring that the required force to remove the tracheostomy guard 10 may be relatively large (so as to retain the tracheostomy guard 10 sturdily in use), while making it relatively easy for the user to impose the force required to remove the tracheostomy guard 10 from the patient interface 20.

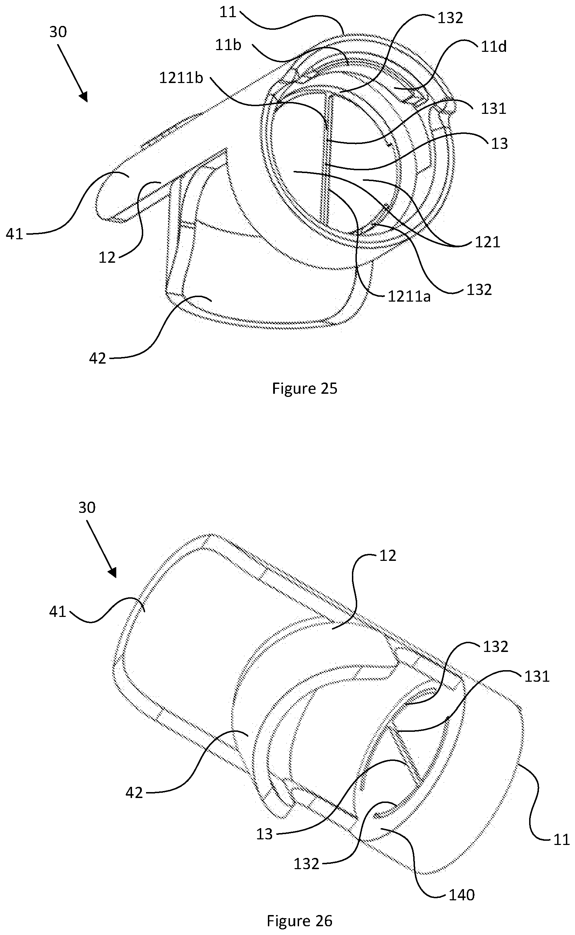

In some embodiments, the attachment member 11b comprises a protrusion extending inwardly towards a longitudinal centre axis of the first portion 11 to mate with a corresponding feature 24, such as a recess, of the patient interface 20. Embodiments showing this feature are shown with reference to Figures 23 to 30, such as the protrusion 11d shown in Figure 25.

In some alternative embodiments, the attachment member 11b comprises a recess (not shown in the Figures) configured to mate with a corresponding feature such as a protrusion (not shown), of the patient interface 20.

The alignment feature 11a may in some embodiments relate to an exterior shape or boundary of the first portion 11, such as the exterior shape of the edge of the first portion 11. For example, with reference to Figures 2 to 22, the alignment feature 11a of the first portion 11 relates to the exterior shape of an oblong or tongue shaped extension (e.g., attachment release lip 11c) of the first portion 11. The alignment feature 11a may extend in a direction longitudinally away from the second portion 12, and towards the patient interface 20, in use. A portion of this kind may comprise an arcuate shape, a generally rectangular shape, or another shape and may for example, comprise an edge contour that forms at least part of the alignment feature 11a.

Further possible examples of the alignment feature 11a are also shown with reference to Figures 24, 32, 39.

A corresponding alignment feature 25 may be arranged on the patient interface 20. With reference to Figures 13 to 17b, the alignment feature 25 may be formed by one or more protrusions protruding outwardly from the attachment member 221 of the patient interface 20. The alignment feature 25 may have an exterior shape or boundary that aligns with the corresponding shape or boundary of the alignment feature 11a of the tracheostomy guard 10, for example as shown with reference to Figures 15 to 17b.

The protrusion(s) 25 of the patient interface 20 may be symmetrically arranged on either side of the patient interface 20 to align with the corresponding alignment feature(s) 11a of the tracheostomy guard 10. For example, Figure 16 shows the boundary of a first protrusion 25 of the patient interface 20 aligned with the boundary of the right side of the tongue shaped extension 11a, whereas Figured 17a shows the boundary of a second protrusion 25, symmetrically arranged to the first protrusion, aligned with the boundary of the left side of the tongue shaped extension 11a.

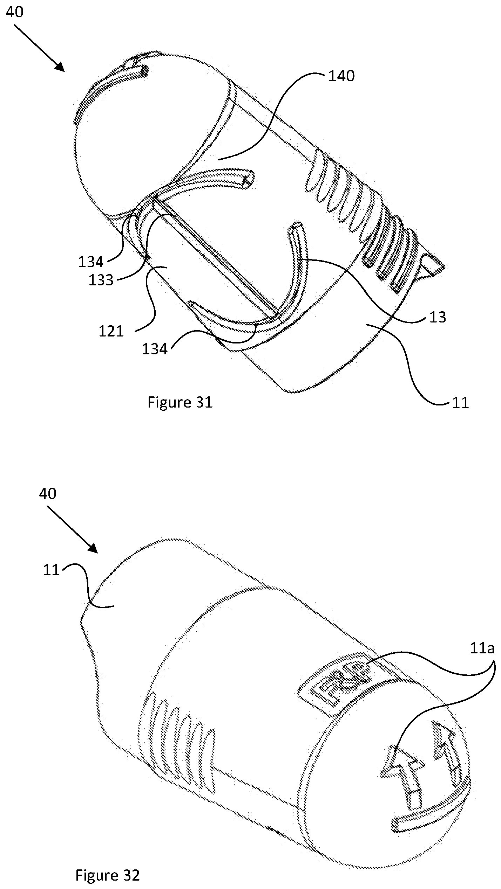

It should be appreciated that in other embodiments the alignment feature 11a may alternatively, or additionally, be referred to, related to, or provided by, the logo best shown in Figures 2, 16, 17a to 17b, 32, 39, 43. In such embodiments, the user may, for example, position the logo in an upward-facing orientation when connecting the tracheostomy guard 10 to the patient interface 20. Additionally or alternatively, the alignment feature 11a may relate to the arrows of Figure 32, ribs of Figure 24. Still other configurations of the alignment feature are within the scope of the invention.

Other configurations of the aperture 13 in the second portion 12 of the tracheostomy guard 10, other than those described above, are within the scope of the invention. In some examples, the second portion 12 may comprise additional (i.e., more than two) members forming or defining the aperture 13. And, while some of the Figures show a singular first member 121, it should be appreciated that the second member 122 and first member 121 could instead be formed from a plurality of elements, for example a plurality of flaps.

For instance, the members may have a "petal" configuration such that, pursuant to an applied force acting on or proximate the members (e.g., acting in an inward or outward direction relative to the second portion 12), they move resiliently inwardly or outwardly away from one another to increase the effective size of the aperture 13. In another example, the first member 121 may be split into sub-members, e.g., two equal sub-members, aka flaps, again achieving a "petal" effect whereby the sub-members move generally inwardly or outwardly upon the application of force to or proximate the flaps. In such configurations the members or sub-members will have an appropriate level of rigidity, enabling sputum to initially be retained in the second portion 12 before the members or sub-members eventually resiliently deform in a controlled manner to release the sputum via the aperture 13, as discussed herein.

Another possible example is that the aperture 13 is formed in the second portion 12 but without being defined by distinct "members" in/on the second portion 12. For instance, the second portion 12 may be provided by a single continuous component that is substantially "thimble-shaped" and the aperture 13 may be provided by a slit or incision, such as a cross-shaped incision, at a distal end or tip of the second portion 12, i.e., the end that is furthest from the first portion 11. The material in the vicinity of the incision will have resiliently deformable properties such that the effective size of the aperture 13 varies depending on force applied to at least part of the second portion 12 (specifically, force applied in the vicinity of the slit or incision), which urges the slit or incision open. In a further variation, instead of the aperture 13 being provided by a simple slit(s) or incision(s) on the surface of the second portion 12, the aperture 13 may have a "cusped" configuration somewhat similar to a prosthetic or bioprosthetic heart valve comprising a plurality of "cusps" or "leaflets" extending in three dimensions, i.e., having a depth and three-dimensional contouring.

Some of the examples of the configuration of the tracheostomy guard discussed above are described in further detail below, with reference to the figures.

Figures 23 to 26 illustrate perspective views of a tracheostomy guard 30 according to a third embodiment. The tracheostomy guard 30 comprises two first members 121 with a variable sized aperture 13 at least partly formed or defined therebetween. Further, the variable sized aperture 13 may be at least partly formed between at least one region 140 of the second portion 12 and the respective first member 121. In this way the region 140 could be said to act similarly to the second member of the first embodiment. The region 140 may be provided by the body of the second portion 12 in the vicinity of the aperture 13.

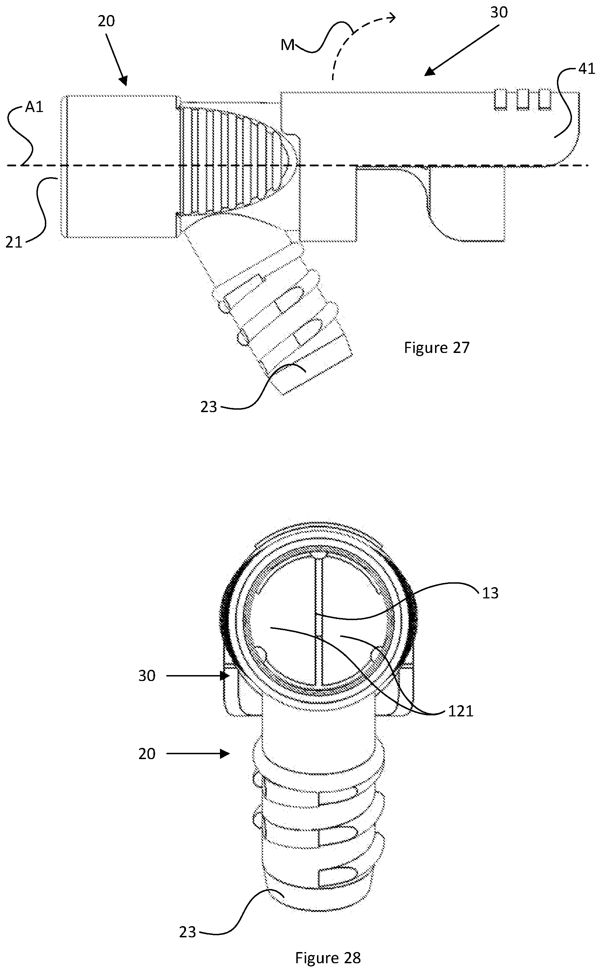

In this embodiment, the two first members 121 are arranged in a plane orthogonal or essentially orthogonal to a longitudinal centre axis of the tracheostomy guard 10. The variable sized aperture 13 is formed or defined between the peripheral ends 1211a, 1211b of each first member 121. In response to the force applied to at least part of the second portion 12, the respective first members 121 may move relative to each other, thus varying the sized of the variable sized aperture 13. The second portion 12 may comprise an optionally curved deflecting terminating member 42 (here shown in conjunction with supporting / reinforcing structure 41) extending at an angle to the longitudinal centre axis, for example perpendicular to the longitudinal centre axis. The deflecting terminating member 42 acts to arrest and / or deflect any sputum or secretions or gases exiting the tracheostomy guard 10 via the variable sized aperture 13, e.g., to prevent them from being ejected in an uncontrolled fashion. As may be observed from Figures 23 to 26, the variable sized aperture 13 may have or be formed by at least one straight extension 131 and one or more curved extensions 132 optionally arranged substantially transverse to the straight extension. Figures 27 to 31 illustrate various views of the tracheostomy guard 30 of the third embodiment when connected to the patient interface 20 of Figures 13 to 14.

In some embodiments the variable sized aperture 13 may in the resting state be arranged on a union surface with one or more curvatures rather than in a union plane as shown with reference to Figures 2 to 30.

Figures 31 to 34 illustrate respective perspective views of a tracheostomy guard 40 according to a fourth embodiment. Like that of the third embodiment, the tracheostomy guard 40 comprises two first members 121 forming at least part of a variable sized aperture 13 provided therebetween.

Like in the third embodiment, the variable sized aperture 13 may be at least partly formed between at least one region 140 of the second portion 12 and the respective first member 121. The region 140 may be said to be provided by the body of the second portion 12 in the vicinity of the aperture 13.

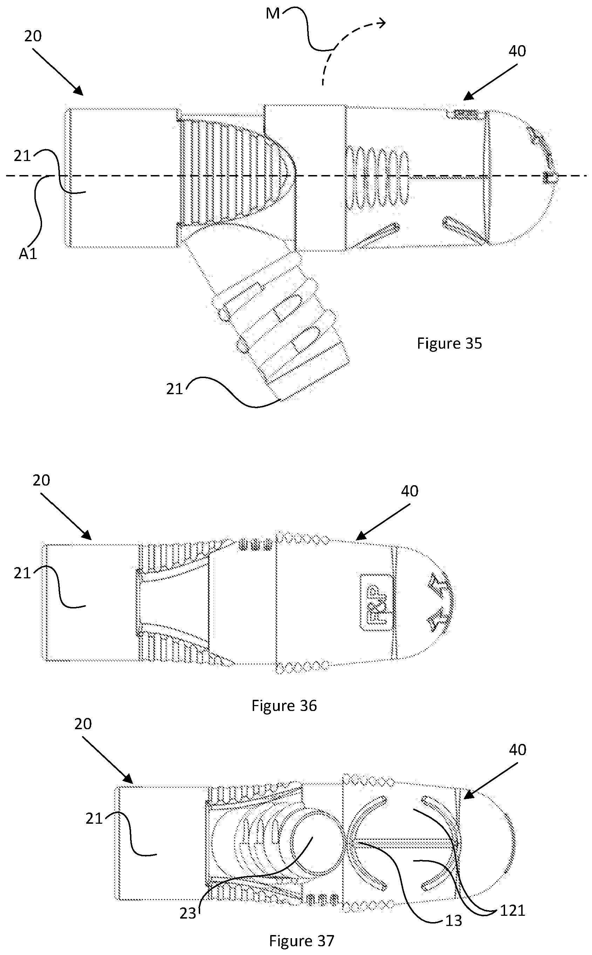

In the fourth embodiment, however, the two first members 121 are arranged at a side of the tracheostomy guard 10, such as the bottom side in use, to make use of the gravitational forces acting on any sputum or secretions present in the second portion 12 and/or such that deflection of the two first members 121 is assisted by gravity when sputum deposits are present on the two first members 121. In this embodiment, the variable sized aperture 13 is formed or defined between the peripheral ends 1211a, 1211b of each first member 121. In response to the force applied to at least part of the second portion 12, the respective first members 121 may move relative each other, thus varying the size of the variable sized aperture 13. As may be observed from Figures 32 to 34, the variable sized aperture 13 may have at least one straight extension 133 arranged parallel or substantially parallel to the longitudinal centre axis of the tracheostomy guard 10. The variable sized aperture 13 may further have one or more curved extensions 134 optionally arranged substantially transverse to the straight extension 133. Figures 35 to 37 illustrate various views of the tracheostomy guard 40 of the fourth embodiment when connected to the patient interface of Figures 13 to 14.

Figures 38 to 42 illustrate respective perspective views of a tracheostomy guard 50 according to a fifth embodiment. In this embodiment the tracheostomy guard 50 comprises two sets of four first members 121a-d, arranged on opposite sides of the tracheostomy guard 50. The opposite sides may be referred to as top and bottom sides, or non-lateral sides, in relation to the intended orientation of the tracheostomy guard 50 in use. Each set of first members 121a-d are arranged to form a respective variable sized aperture 13 therebetween.

Similar to the second and third embodiment, at least part of the variable sized aperture 13 may in addition be formed between at least one region 140 of the second portion 12 and the respective first member 121a-d. In this way the region 140 proximate to the aperture 13 could be said to act similarly to the second member of the first embodiment.

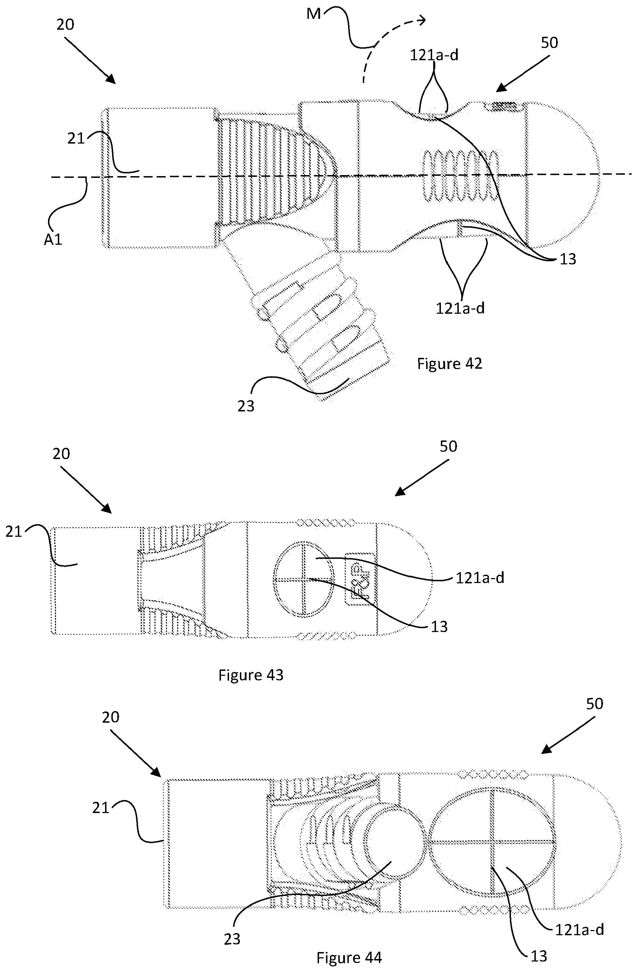

The respective variable sized aperture 13 of the tracheostomy guard 50 may form a cross shape with two crossing straight extensions. Each variable sized aperture 13 is formed or defined by the peripheral ends 1211a of the respective first member 121 of each set and has a size that varies in response to a force applied to at least part of the second portion 12, in use. With two variable sized apertures 13 one may act as a safety backup. As previously explained when the first member 121 is arranged on the bottom side (in terms of its intended orientation in use) it may advantageously make use of the gravitational forces acting on any sputum or secretions present in the second portion 12. In response to the force applied to at least part of the second portion 12, the set of first members 121a-121d may move relative to each other, thus varying the size of the associated variable sized aperture 13. Figures 42 to 44 illustrate various views of the tracheostomy guard 50 of the fifth embodiment when connected to the patient interface of Figures 13 to 14.

In an alternative embodiment, rather than the first member 121 being formed from a plurality of flaps, the portion of the first member 121 at a distal end may be segmented thereby enabling discrete and localised deformation of a section of the first member 121 owing to flow or otherwise.

According to a further embodiment, a valve unit is provided. The valve unit comprises a hollow body having a first port for connection to a corresponding port of a tracheostomy guard and a second port in fluid communication with the ambient environment. The valve unit further comprises at least one first member 121 and a second member 122 forming at least one aperture having an effective size that is variable in response to a force applied to the first member and/or second member 122, in use. The first member 121 and/or second member 122 may be attached to the body of the valve unit. The variable sized aperture may, in a resting state, be arranged at the first port, at the second port, or between the first and second ports.

According to an alternative embodiment, an insert, or valve component for fitting or retrofitting to an existing tracheostomy guard is provided. The insert or valve component may be configured to facilitate an aperture having a variable size in use in response to applied force. An example of an existing tracheostomy guard is the sputum guard (OPT971) used / sold in conjunction with the Optiflow+ Tracheostomy interface (OPT970). The OPT971 sputum guard attaches to the OPT970 interface, and has an upper and lower rigid aperture. It is within the scope of the present invention to provide an insert, or valve component, for the OPT971 sputum guard, said insert comprising the variable-size aperture(s) as herein described. For example, the insert could be formed from an appropriately flexible material, and could have a configuration substantially complementary to that of the OPT971 sputum guard so as to substantially mate with the OPT971 sputum guard, for instance sit snugly within (or even outside of) the OPT971 sputum guard. The insert would comprise the variable-size apertures of the present invention (for example provided by a slit(s) in the flexible material), positioned to coincide with the rigid apertures of the OPT971 sputum guard. Likewise, with appropriate modifications, such an insert or valve component could be provided for fitting or retrofitting to other types, brands or models tracheostomy guards.

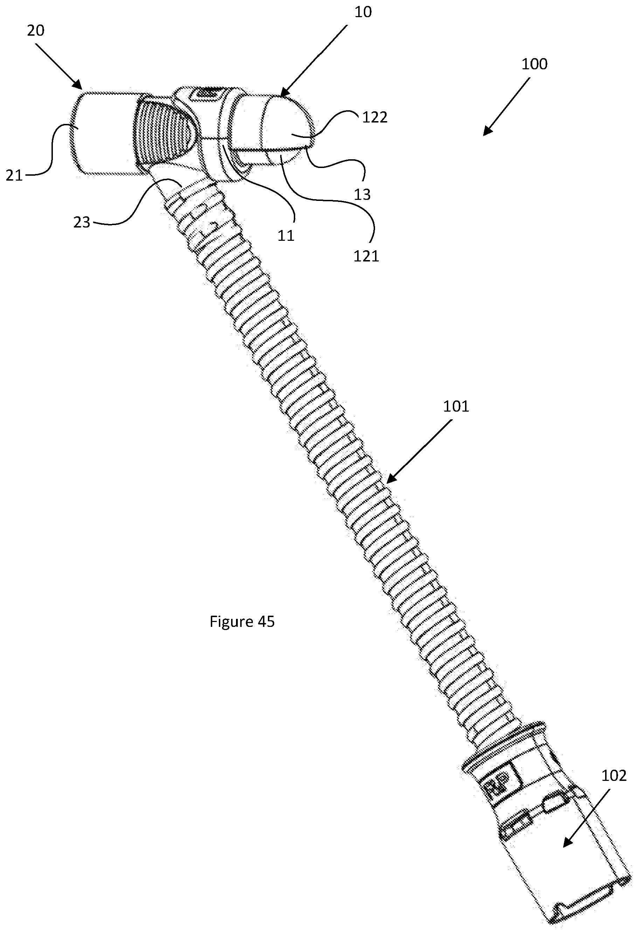

Figure 45 illustrates perspective view of an assembly 100 for a respiratory support system comprising the tracheostomy guard 10 of the first embodiment. The assembly 100 further comprises a tracheostomy patient interface 20 for connection to the tracheostomy guard 10, and a supply tube or conduit 101 for connection to the port 23 of the patient interface 20 for providing a gases flow from a gases source (not shown).

The supply tube or conduit 101 may have a connector 102 for connection to the gases source.

The supply tube or conduit 101 may primarily comprise an inlet, an outlet, and an enclosing wall defining a gases passageway between said inlet and said outlet. At least a region of said wall comprises a membrane that is of a breathable material to allow the passage of water vapour without allowing the passage of liquid water or respiratory gases. Preferably substantially the entire length of the supply tube or conduit 101 is configured to allow the passage of water vapour without allowing the passage of liquid water or respiratory gases.

The tracheostomy guard according to the present invention may be provided in a kit of parts that can comprise one or more of: the tracheal coupling (e.g., the tracheostomy guard 10, 30, 40 or 50); the tracheostomy patient interface 20; the valve component configured to fit to the tracheal coupling; the tracheal tube coupled or couplable to the tracheostomy patient interface 20 and/or configured to fluidly connect the tracheostomy patient interface 20 with the airway of a patient; a connector for connecting the tracheal tube to the tracheostomy patient interface 20; the conduit or tube arranged to connect the tracheostomy patient interface 20 to a gases source; and the gases source.

In one example, there is provided a kit of parts comprising the tracheostomy guard 10, 30, 40 or 50 and any one or more of: the patient interface 20; the tracheal tube; the connector for connecting the tracheal tube to the tracheostomy patient interface 20; the conduit or tube arranged to connect the tracheostomy patient interface 20 to a gases source; and the gases source.

In another example, there is provided a kit of parts comprising the valve component and any one or more of: the tracheal coupling (adapted to receive the valve component); the patient interface 20; the tracheal tube; the connector for connecting the tracheal tube to the tracheostomy patient interface 20; the conduit or tube arranged to connect the tracheostomy patient interface 20 to a gases source; and the gases source.

It should be appreciated that at least some parts of the kit can be pre-assembled (e.g., the tracheostomy guard 10 and the tracheostomy patient interface 20 may be pre-assembled together) for easier packaging and/or installation when being used for the first time.

As used herein, the term "breathable" generally means highly permeable to water vapor and substantially impermeable to liquid water and the bulk flow of gases. A "breathable material" as used herein generally refers to a material that is highly permeable to water vapor and substantially impermeable to liquid water and the bulk flow of gases.

The supply tube or conduit 101 may include at least one helically wound polymer tape or strip, part or all of said strip comprising the membrane, respective edges of adjacent turns of said strip being adjoining or overlapping and bonded to form the enclosing wall.

One possible material for the breathable regions is an activated perfluorinated polymer material having extreme hydrophilic properties. An example of this polymer material is marketed under the trade name NAFION^{®} by DuPont Fluoro products of Fayetteville USA. This material is useful due to its extreme hydrophilic properties and due to its ability to be extruded, particularly to be co-extruded in combination with other plastic materials.

Alternative materials include:
(a) Hydrophilic thermoplastics,
(b) Woven treated fabric products exhibiting breathable characteristics.

A particularly suitable material is a hydrophilic polyester block copolymer formed into a homogeneous flat film. An example of such a film is sold under the brand SYMPATEX^{®}. This material is particularly suited to thin film productions.

The supply tube or conduit 101 may include lateral reinforcement against deformation of the breathing gas conduit, such as a helical bead disposed over said adjoining or overlapping edges between turns of strip, or a series of annular ring beads or ribs distributed over the length of said conduit. The bead may be formed from a thermoplastic, and is preferably made of a polyester-based polymer. The tape or strip and bead may both be polyester based polymers, which improves the bond between them. The bead may be made of a material sold under the trade name Arnitel^{®} EM550.

The supply tube or conduit 101 may further or alternatively include longitudinal reinforcement against stretching of the supply tube or conduit 101.

The gases source may for example be a flow generator, ventilator, humidifier, or any other gases source for supplying a gases flow rate to the patient interface. In some embodiments the assembly 100 comprises the gases source.

While the assembly 100 of Fig 45 is shown with reference to the tracheostomy guard 10 according to the first embodiment, it should be appreciated that the assembly 100 may also comprise the tracheostomy guard according to any of the embodiments disclosed herein.

## Claims

1. A tracheostomy guard (10, 30, 40, 50) for secretion removal from a patient interface (20), comprising:
a first portion (11) arranged to connect to a patient interface (20) in fluid communication with the first portion (11), in use,
a second portion (12) in fluid communication with the first portion (11), comprising at least one first member (121) and/or second member (122) forming an aperture (13) in fluid communication with the ambient environment,
wherein:
the aperture (13) has a size variable in response to a force applied to at least part of the second portion (12), in use; and
the aperture (13) remains open regardless of the magnitude or extent of the applied force.

2. The tracheostomy guard (10, 30, 40, 50) according to claim 1, wherein the at least one first member (121) is arranged to deflect or move relative to the second member (122) in response to the force applied.

3. The tracheostomy guard (10, 30, 40, 50) according to claim 1 or 2, wherein the size of the aperture (13) relates to a cross-sectional dimension of the aperture (13).

4. The tracheostomy guard (10, 30, 40, 50) according to any one of the preceding claims, wherein the variable size of the aperture (13) is configured to allow for sputum release therethrough in response to the force applied, in use.

5. The tracheostomy guard (10, 30, 40, 50) according to any one of the preceding claims, wherein the force applied to the second portion (12) in use is due at least part to a weight of sputum present in the second portion (12).

6. The tracheostomy guard (10, 30, 40, 50) according to any one of the preceding claims, wherein the variable size of the aperture (13) is configured to achieve a set minimum positive end expiratory pressure (PEEP), in use.

7. The tracheostomy guard according to claim 6, wherein set minimum PEEP is selected from a range of: 1cmH₂0 to 7cmH₂0.

8. The tracheostomy guard (10, 30, 40, 50) according to any one of the preceding claims, wherein the at least one first member (121) and/or the second member (122) are relatively arranged to form the variable sized aperture (13).

9. The tracheostomy guard (10, 30, 40, 50) according to any of the preceding claims, wherein the first member(s) (121) has a first material or structural characteristic, and the second member(s) (122) has a second material or structural characteristic, wherein the first material or structural characteristic and the second material or structural characteristic are selected to allow the size of the variable sized aperture (13) to change in response to the applied force.

10. The tracheostomy guard (10, 30, 40, 50) according to any one of the preceding claims, wherein the at least one first member (121) or second member (122) comprises a flap or panel.

11. The tracheostomy guard of any one of the preceding claims, wherein said second portion (12) is configured to be in fluid communication with the patient interface (20), in use.

12. The tracheostomy guard (10, 30, 40, 50) according to claim 2, wherein the movement of the first member (121) relative to the second member (122) is bi-directional.

13. The tracheostomy guard (10, 30, 40, 50) according to any preceding claim, wherein the first member (121) or second member (122) is cantilevered off the first portion (11), or the first member (121) is cantilevered off the second member (122), or the second member (122) is cantilevered off the first member (121).

14. The tracheostomy guard (10, 30, 40, 50) according to any preceding claim, wherein the aperture (13) faces downwardly.

## Patentansprüche

1. Tracheostomieschutz (10, 30, 40, 50) zur Entfernung von Sekret aus einer Patientenschnittstelle (20), umfassend:
einen ersten Abschnitt (11), der dazu eingerichtet ist, im Gebrauch an eine Patientenschnittstelle (20) angeschlossen zu werden, wobei die Patientenschnittstelle (20) mit dem ersten Abschnitt (11) in Fluidverbindung steht,
einen zweiten Abschnitt (12), der mit dem ersten Abschnitt (11) in Fluidverbindung steht und mindestens ein erstes Element (121) und/oder ein zweites Element (122) umfasst, das beziehungsweise die eine Öffnung (13) bildet beziehungsweise bilden, die mit der Umgebung in Fluidverbindung steht,
wobei:
die Öffnung (13) eine Größe aufweist, die im Gebrauch als Reaktion auf eine auf mindestens einen Teil des zweiten Abschnitts (12) ausgeübte Kraft variabel ist; und
die Öffnung (13) unabhängig vom Betrag oder Ausmaß der ausgeübten Kraft offen bleibt.

2. Tracheostomieschutz (10, 30, 40, 50) nach Anspruch 1, wobei das mindestens eine erste Element (121) dazu eingerichtet ist, sich als Reaktion auf die ausgeübte Kraft relativ zu dem zweiten Element (122) auszulenken oder zu bewegen.

3. Tracheostomieschutz (10, 30, 40, 50) nach Anspruch 1 oder 2, wobei sich die Größe der Öffnung (13) auf eine Querschnittsabmessung der Öffnung (13) bezieht.

4. Tracheostomieschutz (10, 30, 40, 50) nach einem der vorhergehenden Ansprüche, wobei die variable Größe der Öffnung (13) dazu eingerichtet ist, im Gebrauch als Reaktion auf die ausgeübte Kraft die Freisetzung von Sputum durch die Öffnung hindurch zu ermöglichen.

5. Tracheostomieschutz (10, 30, 40, 50) nach einem der vorhergehenden Ansprüche, wobei die im Gebrauch auf den zweiten Abschnitt (12) ausgeübte Kraft zumindest teilweise durch das Gewicht des im zweiten Abschnitt (12) vorhandenen Sputums bedingt ist.

6. Tracheostomieschutz (10, 30, 40, 50) nach einem der vorhergehenden Ansprüche, wobei die variable Größe der Öffnung (13) dazu eingerichtet ist, im Gebrauch einen festgelegten Mindestwert des positiven endexspiratorischen Drucks (PEEP) zu erreichen.

7. Tracheostomieschutz nach Anspruch 6, wobei der festgelegte Mindestwert des positiven endexspiratorischen Drucks (PEEP) aus einem Bereich von 1 cmH₂O bis 7 cmH₂O ausgewählt ist.

8. Tracheostomieschutz (10, 30, 40, 50) nach einem der vorhergehenden Ansprüche, wobei das mindestens eine erste Element (121) und/oder das zweite Element (122) relativ zueinander angeordnet sind, um die Öffnung (13) mit variabler Größe zu bilden.

9. Tracheostomieschutz (10, 30, 40, 50) nach einem der vorhergehenden Ansprüche, wobei das erste Element beziehungsweise die ersten Elemente (121) eine erste Material- oder Struktureigenschaft aufweist beziehungsweise aufweisen und das zweite Element beziehungsweise die zweiten Elemente (122) eine zweite Material- oder Struktureigenschaft aufweist beziehungsweise aufweisen, wobei die erste Material- oder Struktureigenschaft und die zweite Material- oder Struktureigenschaft so ausgewählt sind, dass sich die Größe der Öffnung (13) mit variabler Größe als Reaktion auf die ausgeübte Kraft ändern kann.

10. Tracheostomieschutz (10, 30, 40, 50) nach einem der vorhergehenden Ansprüche, wobei das mindestens eine erste Element (121) oder das zweite Element (122) eine Klappe oder Platte umfasst.

11. Tracheostomieschutz nach einem der vorhergehenden Ansprüche, wobei der genannte zweite Abschnitt (12) dazu eingerichtet ist, im Gebrauch mit der Patientenschnittstelle (20) in Fluidverbindung zu stehen.

12. Tracheostomieschutz (10, 30, 40, 50) nach Anspruch 2, wobei die Bewegung des ersten Elements (121) relativ zu dem zweiten Element (122) bidirektional ist.

13. Tracheostomieschutz (10, 30, 40, 50) nach einem der vorhergehenden Ansprüche, wobei das erste Element (121) oder das zweite Element (122) von dem ersten Abschnitt (11) oder dem zweiten Abschnitt (12) auskragend angeordnet ist, oder das erste Element (121) von dem zweiten Element (122) auskragend angeordnet ist, oder das zweite Element (122) von dem ersten Element (121) auskragend angeordnet ist.

14. Tracheostomieschutz (10, 30, 40, 50) nach einem der vorhergehenden Ansprüche, wobei die Öffnung (13) nach unten weist.

## Revendications

1. Protection pour trachéotomie (10, 30, 40, 50) pour l'élimination de sécrétions d'une interface patient (20), comprenant :
une première portion (11) agencée pour se raccorder à une interface patient (20) en communication fluidique avec la première portion (11), en utilisation,
une deuxième portion (12) en communication fluidique avec la première portion (11), comprenant au moins un premier organe (121) et/ou un deuxième organe (122) formant une ouverture (13) en communication fluidique avec l'environnement ambiant,
dans lequel :
l'ouverture (13) possède une taille variable en réponse à une force appliquée à au moins une partie de la deuxième portion (12), en utilisation ; et
l'ouverture (13) reste ouverte quelle que soit l'amplitude ou le degré de la force appliquée.

2. Protection pour trachéotomie (10, 30, 40, 50) selon la revendication 1, dans lequel l'au moins un premier organe (121) est agencé pour fléchir ou se déplacer par rapport au deuxième organe (122) en réponse à la force appliquée.

3. Protection pour trachéotomie (10, 30, 40, 50) selon la revendication 1 ou 2, dans lequel la taille de l'ouverture (13) se rapporte à une dimension de section transversale de l'ouverture (13).

4. Protection pour trachéotomie (10, 30, 40, 50) selon l'une quelconque des revendications précédentes, dans lequel la taille variable de l'ouverture (13) est configurée pour permettre la libération d'expectorations à travers celle-ci en réponse à la force appliquée, en utilisation.

5. Protection pour trachéotomie (10, 30, 40, 50) selon l'une quelconque des revendications précédentes, dans lequel la force appliquée à la deuxième portion (12) en utilisation est due au moins en partie à un poids d'expectorations présentes dans la deuxième portion (12).

6. Protection pour trachéotomie (10, 30, 40, 50) selon l'une quelconque des revendications précédentes, dans lequel la taille variable de l'ouverture (13) est configurée pour atteindre une pression d'expiration finale positive (PEEP) minimale de consigne, en utilisation.

7. Protection pour trachéotomie selon la revendication 6, dans lequel la PEEP minimale de consigne est sélectionnée parmi une plage de : 1 cmH₂O à 7 cmH₂O.

8. Protection pour trachéotomie (10, 30, 40, 50) selon l'une quelconque des revendications précédentes, dans lequel l'au moins un premier organe (121) et/ou le deuxième organe (122) sont agencés l'un par rapport à l'autre pour former l'ouverture (13) de taille variable.

9. Protection pour trachéotomie (10, 30, 40, 50) selon l'une quelconque des revendications précédentes, dans lequel le(s) premier(s) organe(s) (121) comporte(nt) une première caractéristique matérielle ou structurelle, et le(s) deuxième(s) organe(s) (122) comporte(nt) une deuxième caractéristique matérielle ou structurelle, dans lequel la première caractéristique matérielle ou structurelle et la deuxième caractéristique matérielle ou structurelle sont sélectionnées pour permettre à la taille de l'ouverture (13) de taille variable de changer en réponse à la force appliquée.

10. Protection pour trachéotomie (10, 30, 40, 50) selon l'une quelconque des revendications précédentes, dans lequel l'au moins un premier organe (121) ou deuxième organe (122) comprend un clapet ou écran.

11. Protection pour trachéotomie selon l'une quelconque des revendications précédentes, dans lequel ladite deuxième portion (12) est configurée pour être en communication fluidique avec l'interface patient (20), en utilisation.

12. Protection pour trachéotomie (10, 30, 40, 50) selon la revendication 2, dans lequel le mouvement du premier organe (121) par rapport au deuxième organe (122) est bidirectionnel.

13. Protection pour trachéotomie (10, 30, 40, 50) selon une quelconque revendication précédente, dans lequel le premier organe (121) ou deuxième organe (122) est en porte-à-faux par rapport à la première portion (11), ou le premier organe (121) est en porte-à-faux par rapport au deuxième organe (122), ou le deuxième organe (122) est en porte-à-faux par rapport au premier organe (121).

14. Protection pour trachéotomie (10, 30, 40, 50) selon une quelconque revendication précédente, dans lequel l'ouverture (13) fait face vers le bas.
